(19) **Europäisches Patentamt / European Patent Office / Office européen des brevets**

(11) **EP 2 393 469 B1**

(12) **EUROPEAN PATENT SPECIFICATION**

(45) Date of publication and mention
of the grant of the patent:
**12.10.2016 Bulletin 2016/41**

(21) Application number: **10702089.3**

(22) Date of filing: **26.01.2010**

(51) Int Cl.:
*A61Q 5/02* (2006.01)　　*A61K 8/41* (2006.01)
*A61K 8/02* (2006.01)

(86) International application number:
**PCT/EP2010/050849**

(87) International publication number:
**WO 2010/089228 (12.08.2010 Gazette 2010/32)**

(54) **Process of treating hair**

Verfahren zur Behandlung von Haar

Procédé de traitement des cheveux

(84) Designated Contracting States:
**AT BE BG CH CY CZ DE DK EE ES FI FR GB GR
HR HU IE IS IT LI LT LU LV MC MK MT NL NO PL
PT RO SE SI SK SM TR**

(30) Priority: **05.02.2009 EP 09290087**

(43) Date of publication of application:
**14.12.2011 Bulletin 2011/50**

(73) Proprietor: **Rhodia Opérations
93306 Aubervilliers (FR)**

(72) Inventors:
• **BENDEJACQ, Denis
F-75005 Paris (FR)**
• **MABILLE, Caroline
F-75004 Paris (FR)**
• **LEROY, Eric
75018 Paris (FR)**

(74) Representative: **Cardon, Flavie
Rhodia Operations
Direction Propriété Industrielle
40, rue de la Haie-Coq
93306 Aubervilliers Cedex (FR)**

(56) References cited:
WO-A1-03/055455　　WO-A2-2006/127394
US-A1- 2006 135 627　　US-A1- 2007 280 976

• RHODIA: "Miracare SLB Structured Surfactant Liquid System" INTERNET ARTICLE, [Online] February 2007 (2007-02), XP002539968 Retrieved from the Internet: URL:http://www.in-cosmetics.com/ExhibitorLibrary/405/Miracare_SLB_Brochure_1.pdf> [retrieved on 2009-08-03]
• DATABASE WPI Week 200215 Thomson Scientific, London, GB; AN 2002-110131 XP002539999 & JP 2001 311099 A (LION CORP) 9 November 2001 (2001-11-09)
• DATABASE WPI Week 200473 Thomson Scientific, London, GB; AN 2004-740994 XP002540070 & JP 2004 292387 A (LION CORP) 21 October 2004 (2004-10-21)

**Description**

FIELD OF THE INVENTION

The present invention concerns a process for improving hair treatment by the deposition of an oil onto the hair.

BACKGROUND OF THE INVENTION

Shampoo compositions are designed to provide certain benefits, including cleaning. These are usually aqueous compositions. Conditioning compositions are designed to provide some further treatment of hair. Some compositions are designed to provide both cleaning and treatment benefits. These are often referred to as 2-in-1 (cleaning and conditioning) shampoos. Some shampoo compositions are designed to provide benefits on virgin hair and/or on damaged hair.

Various compositions have been disclosed in the literature and/or have been made available to consumers. Shampoos typically comprise surfactants, usually anionic surfactants, in the form of micellar aqueous solutions. Using silicones in shampoo compositions is known. Using cationic polymers in shampoos is also known. It is believed that some silicone oil deposits onto hair to provide hair treatment. It is believed that cationic polymers can assist the deposition of silicone oil.

Using cationic surfactants in conditioners to provide hair treatment benefits is known.

Recently cosmetic products, especially skin cleansing products such as body wash products, comprising structured surfactant systems have been developed and commercialized. These cosmetic products are able to suspend high loads of oil that can treat skin, and present interesting rheology and/or aspect and/or sensorial profile. The structured surfactants systems are typically based on an appropriate combination of anionic surfactants and structurants. Such cosmetic products are for example described in documents EP586275 and WO 03/055456.

There is a need for improved aqueous compositions, suitable as shampoo, especially as 2-in-1 shampoos, that provide improved treatment of hair, especially of damaged hair. When hair comprises damaged hair and virgin hair, there is a need for compositions that target damaged hair, to provide more improvement to damaged hair than to virgin hair.

BRIEF SUMMARY OF THE INVENTION

The invention is set out in the claims.

The composition allows an improved treatment of damaged hair. The invention also allows a improved selectivity of treatment to damaged hair compared to virgin hair. The invention also allows targeting damaged hair. The invention also allows an improved ratio of:

treatment of damaged hair / treatment of virgin hair.

The improved treatment of damaged hair and/or selectivity or and/or ratio can allow avoiding large quantities of composition. It can allow lowering the frequency of the use of the composition. It can allow avoiding build-up deposition, especially on virgin hair where not much treatment is needed.

It has been found that the introduction of cationic surfactants into typical compositions comprising surfactants in micellar form decreases the oil treatment, whereas the oil treatment is surprisingly increased when the composition comprises a structured surfactant system. It has been found that the introduction of a cationic surfactant in compositions comprising a structured surfactant system surprisingly increases the selectivity of treatment to damaged hair.

The invention also concerns a process of preparing the composition. The invention also concerns the use of the composition to treat hair, especially damaged hair. The invention also concerns a process for improving hair treatment by deposition of an oil onto hair comprising the step of applying the composition onto hair.

DETAILED DESCRIPTION OF THE INVENTION

The aqueous composition of the invention comprises various ingredients that are detailed below.

In the present applications, unless otherwise provided, amounts are expressed a active % by weight, as opposed to amounts "as is".

a) Structure Surfactant system

The structured surfactant system is a mixture of components that include a surfactant component, and optionally other components such as an electrolyte. The components and the amounts thereof are such that the system forms ordered liquid crystals in the composition. As the composition comprises a structured surfactant system it can also be referred to below as a "Structure surfactant composition". The structured surfactant system is able to impart some specific properties to the composition (in the presence and/or in the absence of further ingredients such as b) and/or c), at same active content), some of which are discussed below and can be considered as a signature of the presence of a structured surfactant system.

Structured surfactant compositions are liquid crystalline compositions, having an ordered liquid crystal structure. Surfactants in the structured surfactant compositions exist in the form of lamellar surfactant phases that are planar and/or in the form of spherulites (also referred to as multilamellar vesicles). Commonly, the surfactant phase is present as spherulites, i.e., lamellar droplets, dispersed in the aqueous phase. Spherulites consist of an onion-like configuration of concentric bi-layers of surfactant molecules, between which is trapped water or electrolyte solution. Exclusively planar lamellar surfactant phases or exclusively spherulite lamellar surfactant phases or the combination of both forms can co-exist in the same composition. Structured surfactant compositions are typically pumpable, non-Newtonian compositions that have the capacity physically to suspend water insoluble particles by virtue of the presence of these lamellar surfactant phases. As referred to herein, "lamellar surfactant phases" are phases which comprise a plurality of stacked bilayers of surfactant separated by a liquid medium. Lamellar phases are typically pourable, non-Newtonian, anisotropic compositions that are cloudy looking and exhibit a characteristic "smeary" appearance on flowing. Lamellar phases can exist in several different forms, including planar layers of parallel sheets, each sheet of which is a bilayer of surfactant, and spherulites formed from layers of concentric spherical shells, each shell of which is a bilayer of surfactant. The spherulites are typically between 0.1 and 50 microns in diameter and so differ fundamentally from micelles. Thus the structured surfactant system can typically present the following:

- planar lamellar phases,
- multilamellar vesicles phases, or
- a mixture of planar lamellar and multilamellar vesicles phases.

In one embodiment, the presence of a surfactant phase having an ordered liquid crystal structure is indirectly demonstrated by showing that the composition (or the structured surfactant system) has an opaque visual appearance and a yield strength of greater than 0 Pascal. Thus the structured surfactant system and/or the composition can have an opaque visual appearance and exhibit a yield strength of greater than 0 Pa.

In one embodiment, the composition exhibits shear thinning viscosity. In one embodiment, the composition is capable of suspending insoluble or partially insoluble components.

In many cases, it is possible to directly demonstrate the presence of an ordered liquid crystal surfactant phase (typical of a structured surfactant system) by the technique of "freeze-fracture microscopy" in which sample of the composition is frozen by exposure to low temperature, the frozen sample is fractured, and one or more of the fracture surfaces of the fractured frozen sample are examined under a microscope.

It is also possible to indirectly demonstrate the presence of an ordered liquid crystal surfactant phase (or to demonstrate that the surfactants system is a structured one) through inference based on the unique combination of properties exhibited by a composition that comprises such a phase or system.

Due to the presence of the ordered liquid crystal surfactant phase, the composition of the present invention exhibits, on visual inspection, an opaque appearance. The composition of the present invention exhibits an opaque appearance in the absence, as well as in the presence, of the oil. As used herein, the term "opaque" means not completely transparent to light and ranges from a hazy translucent appearance through a turbid appearance to a uniform, saturated white appearance. In one embodiment, the structured surfactant system provides ranges from a turbid appearance to a uniform, saturated white appearance.

Due to the presence of the ordered liquid crystal surfactant phase, the composition of the present invention exhibits a yield strength of greater than 0 Pascal. As used herein, "yield strength" refers to the magnitude of the applied force required to induce the composition to flow. In one embodiment, the composition exhibits a yield strength of greater than 0.1 Pascal ("Pa"), more typically from about 1 to about 100 Pa, and even more typically from about 1 to about 10 Pa, as determined by measurements using a controlled stress/strain rheometer at two or more shear rates. The presence or absence of a non-zero yield strength may also be reliably determined on a qualitative basis by visual observation of the flow characteristics of the composition and the resistance of the composition to deformation caused by, for example, movement of a hand-held spatula a sample of the composition. In one embodiment, the structured surfactant system in the composition is capable of allowing the composition to suspending water insoluble or partially water-soluble components, such as the oil, preferably without having to incorporate suspending / thickening agents such as polymers (typically polyacrylates) or EGDS. The ability to suspend such components may be viewed as one manifestation of the presence of a non-zero yield strength. As used herein, characterization of an aqueous composition as "capable of suspending", or as being "able of suspend" water insoluble or partially water-soluble components means that the composition substantially resists flotation of such components in the composition or sinking of such components in such composition so that such components appear to be neutrally buoyant in such composition and remain at least substantially suspended in such composition under the anticipated processing, storage, and use conditions for such aqueous composition. The ability to suspend water insoluble or partially water-soluble components is one manifestation of the non-zero yield strength of the present invention, that is, the resistance of the structured surfactant composition of the present invention to deformation at low stresses is sufficient to balance the gravitational forces acting on water insoluble or partially water-soluble components, so that the components remain suspended in the structured surfactant composition.

As discussed above, the ordered liquid crystal phase provides to the composition a rheology which is sufficient, when the system is at rest, to immobilize any suspended particles but, upon application of a shearing force, is sufficiently low to allow the system to be pumped like a normal liquid. Such systems may display very low apparent viscosities when stirred, pumped or poured and yet be capable of maintaining particles, sometimes of millimeter or larger size, in suspension. In one embodiment, the structured surfactant system allows the composition to suspend air bubbles for at least 1 week, and more typically for at least 3 months. A composition that is capable of suspending air bubbles for at least 12 hours at room temperature is deemed to be generally capable of suspending water insoluble or partially water-soluble components in the composition under generally anticipated processing, storage, and use conditions for such composition. For components other than air, the result of the air suspension test should be confirmed by conducting an analogous suspension test using the component of interest. For unusually rigorous processing, storage and/or use conditions, more rigorous testing may be appropriate. In one embodiment, the ability to suspend water insoluble or partially water-soluble components is evaluated under more rigorous conditions, that is, the mixed samples are visually evaluated after subjecting the samples to one or more freeze/thaw cycles, wherein each freeze/thaw cycle consists of 12 hours at -10°C and 12 hours at 25°C. In one embodiment, the structured surfactant system allows the composition to remain capable of suspending air bubbles after one freeze/thaw cycle, more typically after 3 freeze/thaw cycles.

In one embodiment, the composition of the present invention exhibits shear-thinning viscosity. As used herein in reference to viscosity, the terminology "shear-thinning" means that such viscosity decreases with an increase in shear rate. Shear-thinning may be characterized as a "non-Newtonian" behavior, in that it differs from that of a classical Newtonian fluid, for example, water, in which viscosity is not dependent on shear rate.

Structured surfactant systems are known by the one skilled in the art, and many have been described in the literature. In many instances the structure is formed when some surfactants (including associations thereof) are associated with a structurant and/or an electrolyte. Structurants and/or electrolytes are known by the one skilled in the art. In the present application they are considered, if present, as part of the structured surfactant system as they typically participate in forming the structure, even if they are not surfactants. In some instances the structure is formed when applying a shear during preparation of the composition and/or during preparation of a premix use for making the composition.

Some surfactant blends that can participate in forming structured domains are commercially available, with reference in the associated documentation to such a property and/or use. Examples include Miracare® SLB grades sold by Rhodia.

Some appropriate structured surfactant systems are also described in the following documents: WO9705857, WO2000059454, WO01019949, WO9932069, WO0170926, WO0170193, WO0267892, WO03017968, WO2005084614, WO2002005758, WO2005063174, WO2005110355, US20080153730, EP586275, WO03055456, WO03055455, WO2006023548, WO2006127394, WO20060135627, WO2008039440, US20080233061.

According to a preferred embodiment, the structured surfactant system a) comprises:

   a1) at least one cationic co-surfactant,
   a2) at least one anionic surfactant,
   a3) optionally at least one structurant,
   a4) optionally at least one amphoteric or zwitterionic surfactant, and
   a5) optionally at least one electrolyte.

According to a preferred embodiment, the structured surfactant system a) comprises:

   a1) at least one cationic co-surfactant,
   a2) at least one anionic surfactant,
   a3) at least one structurant,
   a4) optionally at least one amphoteric or zwitterionic surfactant, and
   a5) optionally at least one electrolyte.

According to a preferred embodiment, the structured surfactant system a) comprises:

   a1) at least one cationic co-surfactant,

a2) at least one anionic surfactant,
a3) at least one structurant,
a4) at least one amphoteric or zwitterionic surfactant, and
a5) at least one electrolyte.

In some embodiments the weight ratio between the cationic co-surfactant and the other surfactant(s) (including structurant(s)) is of from 0.5/100 to 10/100, preferably of from 1/100 to 10/100, preferably of from 2/100 to 8/100.

The natures and amounts of a2), a3), a4), a5) can be set to obtain the structured form. The nature and amounts of a1) a2), a3), a4), a5) can be set to obtain the structured form. Some appropriate natures and amounts are described in the above mentioned documents. Surprisingly it has been found that the introduction of the cationic co-surfactant can allow a structured form while decreasing the amount electrolyte. One can thus surprisingly decrease the amount of electrolyte such as NaCl while adding some cationic co-surfactant, in lesser amount than the amount of subtracted electrolyte. This can allow reducing the amount of salt in the composition and thus lowering irritancy and/or improving mildness.

The total amount of non cationic surfactant(s) (including structurant(s) if any) in the composition can be typically of from 10% to 25% by weight, preferably of from 13.5% to 22%, for example of from 16.5% to 19.5%.

The total amount of surfactant(s) (including structurant(s) if any and cationic co-surfactant(s)) in the composition can be typically of from 10.01% to 30% by weight, preferably of from 13.6% to 25%, for example of from 17% to 20.5%.

In preferred embodiments the structured surfactant system comprises at least 25% by weight, preferably at least 30%, preferably at least 40%, for example alt least 50%, of anionic surfactant(s).

Examples of useful a2) / a3) / a4) / a5) associations include the following:

- Association 1

    - a2) is an alkylethersulfate or alkylsulfate or a mixture thereof, for example sodium laurylether sulfate or ammonium laurylether sulfate
    - a3) is lauric acid or oleic acid
    - a4) is a betaine, preferably an alkylamidopropyl betaine such as cocoamidopropylbetaine
    - a5) is a salt, for example sodium chloride or ammonium chloride,

- Association 2

    - a2) is an alkylethersulfate or alkylsulfate or a mixture thereof, for exemple sodium trideceth sulfate or ammonium trideceth sulfate
    - a3) is an alkanolamide, such a cocamide MEA, cocamide MIPA or cocamide DEA
    - a4) is an amphoacetate or amphodiacetate, for example lauroamphoacetate or lauroamphodiacetate, or cocoamphoacetate or cocoamphodiacetate,
    - a5) is a salt, for example sodium chloride or ammonium chloride.

The total amount of surfactant(s) (including structurants) can be set to obtain a structured form. Usually higher amounts of surfactant(s) allow obtaining structured forms. The amount of electrolyte can also be set to obtain the structured form. Usually higher amounts of electrolyte(s) allow obtaining structured forms. The ratio of total amount of surfactant(s) to electrolyte can as well be set to obtain the structured form.

The ability of a composition to suspend water insoluble or partially water-soluble components is typically evaluated by mixing the composition with sufficient vigor to entrap air bubbles in the composition and then visually observing whether the air bubbles remain entrapped in the composition for a defined period of time, such as for example, 12 to 24 hours, under defined environmental conditions, such as for example, room temperature. In one embodiment, the composition of the present invention is capable of suspending air bubbles for at least 1 week, and more typically for at least 3 months. A composition that is capable of suspending air bubbles for at least 12 hours at room temperature is deemed to be generally capable of suspending water insoluble or partially water-soluble components in the composition under generally anticipated processing, storage, and use conditions for such composition. For components other than air, the result of the air suspension test should be confirmed by conducting an analogous suspension test using the component of interest. For unusually rigorous processing, storage and/or use conditions, more rigorous testing may be appropriate.

In one embodiment, the ability to suspend water insoluble or partially water-soluble components is evaluated under more rigorous conditions, that is, the mixed samples are visually evaluated after subjecting the samples to one or more freeze/thaw cycles, wherein each freeze/thaw cycle consists of 12 hours at -10°C and 12 hours at 25°C. In one embodiment, composition of the present invention remains capable of suspending air bubbles after one freeze/thaw cycle, more typically after 3 freeze/thaw cycles.

a1) cationic co-surfactant

The surfactant system comprises a cationic surfactant (referred to as "cationic co-surfactant). Cationic surfactants are known by the one skilled in the art. Cationic surfactants include surfactants presenting a cationic group or a group that would be cationic upon protonation, for example in acidic conditions, such a quaternary ammoniums or tertiary amines. Cationic surfactants typically do not present an anionic group or an acidic group (otherwise they would be considered as amphoteric or zwitterionic). Quaternary ammonium surfactants are positively charged and are often referred to as quats. Derivatization groups may be aliphatic and may carry additional substituents. The cationic group of the cationic surfactant is usually associated to an anionic counter anion. Typical counter anions include chloride, bromide, methylsulfate (INCI methosulfate), ethylsulfate (INCI ethosulfate).

Examples of cationic surfactants that can be used include the following (INCI names):

Acetamidoethoxybutyl Trimonium Chloride; Acetamidoethyl PG-Trimonium Chloride ; Almondamidopropalkonium Chloride; Apricotamidopropyl Ethyldimonium Ethosulfate; Babassuamidopropalkonium Chloride; Babassuamidopropyltrimonium Chloride; Babassuamidopropyltrimonium Methosulfate; Behenamidopropyl Dimethylamine Behenate; Behenamidopropyl Dimethylamine Lactate; Behenamidopropyl Ethyldimonium Ethosulfate; Behenamidopropyl Glycerylhydroxypropyldimonium Chloride; Behenamidopropyl PG-Dimonium Chloride; Behenamidopropyltrimonium Methosulfate; Behenoyl PG-Trimonium Chloride; Behentrimonium Chloride; Behentrimonium Methosulfate; Canolamidopropyl Ethyldimonium Ethosulfate; Caprylylalkonium Chloride; C12-18 Dialkyldimonium Chloride; Carpronium Chloride; Ceteardimonium Chloride; Ceteartrimonium Chloride; cetrimonium chloride, cetrimonium bromide, cetrimonium methosulfate, Cetyl Betainate Chloride; Cetyldimethylamine Hydrolyzed Hempseedate; Cetyl Ethyldimonium Ethosulfate; Cetyl Pyrrolidonylmethyl Dimonium Chloride; Cinnamidopropyltrimonium Chloride; C10-40 Isoalkylamidopropylethyldimonium Ethosulfate; Cocamidopropyl Dimethylamine Dihydroxymethylpropionate; Cocamidopropyl Dimethylamine Lactate; Cocamidopropyl Dimethylamine Propionate; Cocamidopropyl Dimethylammonium C8-16 Isoalkylsuccinyl Lactoglobulin Sulfonate; Cocamidopropyl Ethyldimonium Ethosulfate; Cocamidopropyl PG-Dimonium Chloride; Cocamidopropyl PG-Dimonium Chloride Phosphate; Cocamidopropyltrimonium Chloride; Cocoalkonium Chloride; Coco-Ethyldimonium Ethosulfate; Cocotrimonium Chloride; Cocotrimonium Methosulfate; Cocoyl Benzyl Hydroxyethyl Imidazolinium Chloride; Cocoyl Hydroxyethylimidazolinium PG-Chloride Phosphate; Dibehenamidopropyldimethylamine Dilinoleate; Dibehenyl/Diarachidyl Dimonium Chloride; Dibehenyldimonium Chloride; Dibehenyldimonium Methosulfate; Di-C12-15 Alkyl Dimonium Chloride; Di-C12-18 Alkyl Dimonium Chloride; Di-C14-18 Alkyl Dimonium Chloride; Dicapryl/Dicaprylyl Dimonium Chloride; Dicetyldimonium Chloride; Dicocodimonium Chloride; Dicocoylethyl Hydroxyethylmonium Methosulfate; Dicocodimethylamine Dilinoleate; Didecyldimonium Chloride; Diethylaminoethyl Cocoate; Diethylaminoethyl PEG-5 Cocoate; Diethylaminoethyl PEG-5 Laurate; Diethylaminoethyl Stearate; Dihydrogenated Palmoylethyl Hydroxyethylmonium Methosulfate; Dihydrogenated Palmoyl Hydroxyethylmonium Methosulfate; Dihydrogenated Tallowamidoethyl Hydroxyethylmonium Chloride; Dihydrogenated Tallowamidoethyl Hydroxyethylmonium Methosulfate; Dihydrogenated Tallow Benzylmonium Chloride; Dihydrogenated Tallowethyl Hydroxyethylmonium Methosulfate; Dihydrogenated Tallow Hydroxyethylmonium Methosulfate; Dihydrogenated Tallowoylethyl Hydroxyethylmonium Methosulfate; Dihydroxyethylamino Hydroxypropyl Oleate; Dihydroxyethyl Cocamine Dioleate; Dihydroxyethyl Soyamine Dioleate; Dihydroxyethyl Tallowamine Dioleate; Dihydroxyethyl Tallowamine Oleate, Dihydroxypropyl PEG-5 Linoleammonium Chloride; Dihydroxypropyl PEG-10 Stearammonium Chloride; Diisostearamidopropyl Epoxypropylmonium Chloride; Dilaureth-4 Dimonium Chloride; Dilauryl Acetyl Dimonium Chloride; Dilauryldimonium Chloride; Dimer Dilinoleamidopropyl PG-Dimonium Chloride Phosphate; Dimethyl Lauramine Dimer Dilinoleate; Dimethyl Lauramine Isostearate; Dimethyl Lauramine Oleate; DimethylPABAmidopropyl Laurdimonium Tosylate; Dioleoyl Edetolmonium Methosulfate; Dioleoylethyl Hydroxyethylmonium Methosulfate; Dioleoylisopropyl Dimonium Methosulfate; Dipalmitoylethyl Dimonium Chloride; Dipalmitoylethyl Hydroxyethylmoniu Methosulfate; Dipalmoylethyl Hydroxyethylmonium Methosulfate; Dipalmoylisopropyl Dimonium Methosulfate; Di-PEG-2 Soyamine IPDI; Dirapeseedoylethyl Hydroxyethylmonium Methosulfate; Disoyamidoethyl Hydroxyethyl Ammonium Lactate; Disoydimonium Chloride; Disoyoylethyl Hydroxyethylmonium Methosulfate; Distearamidopropylmethylamine; Disteareth-6 Dimonium Chloride; Distearoylethyl Dimonium Chloide; Distearoylethyl Hydroxyethylmonium Methosulfate; Ditallowdimonium Chloride; Ditallowethyl Hydroxyethylmonium Methosulfate; Distearyldimethylamine Dilinoleate; Distearyldimonium Chloride; Distearyl Epoxypropylmonium Chloride; Ditallowamidoethyl Hydroxypropylamine; Ditallowamidoethyl Hydroxypropylmonium Methosulfate; Ditallow Dimonium Cellulose Sulfate; Ditallowoylethyl Hydroxyethylmonium Methosulfate; Ditallowoyl PG-dimonium Chloride; Ditridecyldimonium Chloride; Dodecylbenzyltrimonium Chloride; Dodecylhexadecyltrimonium Chloride; Dodecylxylylditrimonium Chloride; Erucalkonium Chloride; Glyceryhydroxypropyl Laurdimonium Chloride; Glycerylhydroxypropyl Steardimonium Chloride; Hydrogenated Palmtrimonium Chloride; Hydrogenated Tallowalkonium Chloride; Hydrogenated Tallowtrimonium Chloride; Hydroxycetyl Hydroxyethyl Dimonium Chloride; Hydroxyethyl Behenamidopropyl Dimonium Chloride; Hydroxyethyl Cetearamidopropyldimonium Chloride; Hydroxyethyl Cetyldimonium Chloride; Hydroxyethyl Cetyldimonium Phosphate; Hydroxyethyl Erucamidopropyl Dimonium Chloride; Hydroxyethyl Laurdimonium Chloride; Hydroxyethyl Oleyl Dimonium Chloride; Hydroxyethyl Tallowdimonium Chloride; Hydroxypropyl Biscetearyldimonium Chloride; Hydroxypropyl Bisisostearamidopropyldimonium Chloride; Hydroxypropyl Bisoleyldimonium Chloride; Hydroxypropyl Bisstearyldimonium Chloride; Hydroxystearamidopropyl Trimonium Chloride; Hydroxystearamidopropyl Trimonium Methosulfate; Isostearamidopropyl Epoxypropylmorpholinium Chloride; Isostearamidopropyl Ethyldimonium Ethosulfate; Isostearamidopropyl Ethylmorpholinium Ethosulfate; Isostearamidopropyl Laurylacetodimonium Chloride; Isostearamidopropyl PG-Dimonium Chloride; Isostearaminopropalkonium Chloride; Isostearoyl PG-Trimonium Chloride; Isostearyl Behenamidopropyl Betainate Chloride; Isostearyl Benzylimidonium Chloride; Isostearyl Dilinoleamidopropyl Betainate Chloride; Isostearyl Ethyldimonium Chloride; Isostearyl Ethylimidazolinium Ethosulfate; Isostearyl Laurdimonium Chloride; Isostearyl Ricinoleamidopropyl Betainate Chloride; Lauramidopropyl PG-Dimonium Chloride; Lauroyl PG-Trimonium Chloride; Laurtrimonium Bromide; Lauryl Diethylenediaminoglycine; Lauryl Dimethylamine Cyclocarboxypropyloleate; Laurylamine Dipropylenediamine; Lauryl Aminopropylglycine; Lauryl Methyl Gluceth-10 Hydroxypropyldimonium Chloride; Linoleamidopropalkonium Chloride; Linoleamidopropyl Dimethylamine Dimer Dilinoleate; Linoleamidopropyl Ethyldimonium Ethosulfate; Meadowfoamamidopropyl PG-Dimonium Chloride; Methoxycinnamidopropyl C18-22 Alkyldimonium Tosylate; Methoxycinnamidopropyl Laurdimonium Tosylate; Methyleicosamidopropyl Ethyldimonium Ethosulfate; Methyl Hydroxycetyl Glucaminium Lactate; Minkamidopropalkonium Chloride; Minkamidopropyl Ethyldimonium Ethosulfate; Myristamidopropyl PG-Dimonium Chloride Phosphate; Octacosatrimonium Chloride; Octyldodecyltrimonium Chloride; Olealkonium Chloride; Oleamidopropyl Dimethylamine Glycolate; Oleamidopropyl Dimethylamine Lactate; Oleamidopropyl Dimethylamine Propionate; Oleamidopropyl PG-Dimonium Chloride; Oleamine Bishydroxypropyltrimonium Chloride; Oleoyl PG-Trimonium Chloride; Oleyl Epoxypropyldimonium Chloride; Olivamidopropyl Dimethylamine Lactate; Olivamidopropyltrimonium Chloride; Palmamidopropyl Trimonium Methosulfate; Palmitamidopropyltrimonium Chloride; Palmitoyl PG-Trimonium Chloride; Panthenyl Hydroxypropyl Steardimonium Chloride; PEG-3 Diethylenetriamine Dipalmamide; PPG-9 Diethylmonium Chloride; PEG-105 Behenyl Propylenediamine; PEG-2 Dimeadowfoamamidoethylmonium Methosulfate; PEG-3 Dioleoylamidoethylmonium Methosulfate; PEG-3 Disoyoylamidoethylmonium Methosulfate; PEG-3 Distearoylamidoethylmonium Methosulfate; PEG-4 Distearylethonium Ethosulfate; PEG-5 Ditridecylmonium Chloride; PEG-15 Hydrogenated Tallowmonium Chloride; PEG-2 Oleammonium Chloride; PEG-5 Oleammonium Methosulfate; PEG-15 Stearmonium Chloride; PEG-22 Tallow Amine; PEG-30 Tallow Amine; PEG-20 Tallow Ammonium Ethosulfate; PEG-15 Tallow Polyamine; PEG-3 Tallow Propylenedimonium Dimethosulfate; PG-Hydroxyethylcellulose Cocodimonium Chloride; PG-Hydroxyethylcellulose Lauryldimonium Chloride; PG-Hydroxyethylcellulose Stearyldimonium Chloride; Quaternium-8; Quaternium-14; Quaternium-16; Quaternium-22; Quaternium-26; Quaternium-27; Quaternium-33; Quaternium-52; Quaternium-53; Quaternium-56; Quaternium-60; Quaternium-61; Quaternium-63; Quaternium-70; Quaternium-72; Quaternium-75; Quaternium-77; Quaternium-78; Quaternium-80; Quaternium-81; Quaternium-82; Quaternium-83; Quaternium-84; Quaternium-85; Quaternium-86; Quaternium-87; Quaternium-88; Quaternium-89; Quaternium-91; Quaternium-92; Quaternium-93; Ricebranamidopropyl Hydroxyethyl Dimonium Chloride; Ricinoleamidopropyltrimonium Chloride; Ricinoleamidopropyltrimonium Methosulfate; Saffloweramidopropyl Ethyldimonium Ethosulfate; Shea Butteramidopropyltrimonium Chloride; Sodium Cocamidopropyl PG-Dimonium Chloride Phosphate; Sodium Dilinoleamidopropyl PG-Dimonium Chloride Phosphate; Sodium Emuamidopropyl PG-Dimonium Chloride Phosphate; Sodium Grapeseedamidopropyl PG-Dimonium Chloride Phosphate; Sodium Myristoyl Sarcosinate; Sodium Milkamidopropyl PG-Dimonium Chloride Phosphate ; Sodium Oleamidopropyl PG-Dimonium Chloride Phosphate; Sodium Olivamidopropyl PG-Dimonium Chloride Phosphate; Sodium Sunfloweramidopropyl PG-Dimonium Chloride Phosphate; Soy Dihydroxypropyldimonium Glucoside; Soyethyldimonium Ethosulfate; Soytrimonium Chloride; Stearamidoethyl Diethanolamine HCl; Stearamidoethyl Diethylamine; Stearamidoethyl Diethylamine Phosphate; Stearamidopropyl Dimethylamine; Stearamidopropyl Dimethylamine Lactate; Stearamidopropyl Dimethylamine Stearate; Stearamidopropyl Ethyldimonium Ethosulfate; Stearamidopropyl Glycerylhydroxypropyldimonium Chloride; Stearamidopropyl PG-Dimonium Chloride Phosphate; Stearamidopropyl Pyrrolidonylmethyl Dimonium Chloride; Stearamidopropyl Trimonium Methosulfate; Stearoxypropyl Dimethylamine; Stearoxypropyltrimonium Chloride; Stearoyl PG-Trimonium Chloride; Steartrimonium Bromide; Steartrimonium Chloride; Steartrimonium Methosulfate; Steartrimonium Saccharinate; Stearyl Dihydroxypropyldimonium Oligosaccharides; Stearyl Ethylhexyldimonium Chloride; Stearyl Ethylhexyldimonium Methosulfate; Stearylgluconamide Dilaurate; Stearyl Hydroxyethylimidonium Chloride; Stearyl PG-Dimethylamine; Stearyl PG-Dimonium Chloride Phosphate; Sunflowerseedamidopropyl Dimethylamine Lactate; Sunflowerseedamidopropyl Dimethylamine Malate; Sunflowerseedamidopropyl Ethyldimonium Ethosulfate; Sunflowerseedamidopropyl Hydroxyethyldimonium Chloride; Sunflowerseedamidopropyl PG-Dimonium Chloride Phosphate; Tallowtrimonium Chloride; Tocopheryl Ethyl Succinate Ethyldimonium Ethosulfate; Tricetylmonium Chloride; Trigonella Foenum-Graecum Hydroxypropyltrimonium Chloride; Undecylenamidopropyltrimonium Methosulfate; Wheat Germamidopropalkonium Chloride; Wheat Germamidopropyl Epoxypropyldimonium Chloride; Wheatgermamidopropyl Ethyldimonium Ethosulfate.

Some preferred cationic surfactants include the following:

- cetrimonium chloride,
- cetrimonium bromide,
- cetrimonium methosulfate
- Behentrimonium Chloride;
- Behentrimonium Methosulfate
- Stearamidopropyl Dimethylamine
- Isostearamidopropyl Dimethylamine
- Oleamidopropyl Dimethylamine
- Behenamidopropyl Dimethylamine
- Cocamidopropyl Dimethylamine Propionate
- Stearamidopropyl Dimethylamine Lactate
- Stearamidopropyl Morpholine Lactate
- Isostearamidopropyl Mospholine Lactate
- Sunflowerseedamidopropyl Dimethylamine.

The composition can typically comprise from 0.01 to 5 % by weight, prefarbly from 0.1 to 2.5%, preferably from 0.5 to 1% of the cationic co-surfactant.

a2) anionic surfactant

The surfactant system preferably comprises an anionic surfactant or a mixture or association of anionic surfactants. Anionic surfactants are known by the one skilled in the art.

In a most preferred embodiment, the anionic surfactant comprises an alkylether sulfate and/or an alkyl sulfate.

In one embodiment, the alkyl ether sulfate surfactant comprises one or more compounds according to structure (I):

$$R^1\text{-O-}(C_mH_{2m}O)_n\text{-So}_3\text{-}X^+ \qquad (I)$$

wherein

$R^1$ is $(C_8\text{-}C_{18})$alkyl or $(C_8\text{-}C_{18})$alkenyl, more typically $(C_{10}\text{-}C_{14})$alkyl,
m is 2, 3, or 4,
n is an integer of from 1 to about 7, more typically from 1 to 8, even more typically from 1 to 6,

$X^+$ is a cation.

In one embodiment, $R^1$ is a branched $(C_8\text{-}C_{18})$alkyl group or a $(C_8\text{-}C_{18})$alkenyl group, more typically a branched $(C_{10}\text{-}C_{16})$alkyl group, such as tridecyl.

Suitable branched alkyl groups include methyldecyl groups, methylundecyl groups, methyldodecyl groups, ethyldecyl groups, ethylundecyl groups, and ethyldodecyl groups, such as for example, 1-methyldecyl, 1-methylundecyl, 1-methyldodecyl, 1-ethyldecyl, 1-ethylundecyl, and 1-ethyldodecyl.

In one embodiment, m is 2 or 3, more typically 2. In one embodiment, n is 1, 2, 3, or 4. As used herein, modifying an alkyl or alkenyl group with the suffix "eth" generally indicates the addition of one or more ethylene oxide units, for example, trideceth refers to an ethoxylated tridecyl group, and the suffix "-n", wherein n is an integer, indicates the number of such ethylene oxide units per group, for example "trideceth-3" indicates an ethoxylated tridecyl group with 3 ethylene oxide units per tridecyl group.

In one embodiment, the alkyl ether sulfate surfactant comprises one or more compounds selected from sodium laureth sulfates, potassium laureth sulfates, magnesium laureth sulfates, ammonium laureth sulfates, monoethanolamine laureth sulfates, diethanolamine laureth sulfates, triethanolamine laureth sulfates, sodium trideceth sulfates, magnesium trideceth sulfates, ammonium trideceth sulfates, monoethanolamine trideceth sulfates, diethanolamine trideceth sulfates, and triethanolamine trideceth sulfates. sodium oleth sulfates, potassium oleth sulfates, magnesium oleth sulfates, ammonium oleth sulfates, monoethanolamine oleth sulfates, diethanolamine oleth sulfates, triethanolamine oleth sulfates.

In one embodiment, the alkyl ether sulfate surfactant comprises one or more branched alkylether sulfate selected from sodium trideceth-1 sulfate, potassium trideceth-1 sulfate, and ammonium trideceth-1 sulfate, sodium trideceth-2 sulfate, potassium trideceth-2 sulfate, and ammonium trideceth-2 sulfate, sodium trideceth-3 sulfate, potassium trideceth-3 sulfate, and ammonium trideceth-3 sulfate, sodium trideceth-4 sulfate, potassium trideceth-4 sulfate, and ammonium trideceth-4 sulfate.

In one embodiment, the alkyl sulfate surfactant comprises one or more compounds according to structure (II):

$$R^2\text{-O-SO}_3^-X^+ \qquad (II)$$

wherein:

R$^2$ is (C$_8$-C$_{18}$)alkyl or (C$_8$-C$_{18}$)alkenyl, and
X$^+$ is a cation.

In one embodiment, R$^2$ is dodecyl, tridecyl, or oleyl.

In one embodiment, the alkyl sulfate surfactant comprises one or more compounds selected from sodium lauryl sulfate, potassium lauryl sulfate, magnesium lauryl sulfate, ammonium lauryl sulfate, monoethanolamine lauryl sulfate, diethanolamine lauryl sulfate, triethanolamine lauryl sulfate, sodium tridecyl sulfate, potassium tridecyl sulfate, magnesium tridecyl sulfate, ammonium tridecyl sulfate, monoethanolamine tridecyl sulfate, diethanolamine tridecyl sulfate, triethanolamine tridecyl sulfate, sodium oleyl sulfate, potassium oleyl sulfate, magnesium oleyl sulfate, ammonium oleyl sulfate, monoethanolamine oleyl sulfate, diethanolamine oleyl sulfate, triethanolamine oleyl sulfate.

Further types of anionic surfactants that can be used, alone or combined with the above sulfates, include:

- linear alkylbenzene sulfonates,
- alpha olefin sulfonates,
- paraffin sulfonates,
- alkyl ester sulfonates,
- alkyl sulfonates,
- alkyl alkoxy carboxylates,
- monoalkyl phosphates,
- dialkyl phosphates,
- sarcosinates,
- isethionates,
- taurates

Particular examples of anionic surfactants that can be used include:

ammonium lauryl sulfate, ammonium laureth sulfate, triethanolamine laureth sulfate, monoethanolamine lauryl sulfate, monoethanolamine laureth sulfate, diethanolamine lauryl sulfate, diethanolamine laureth sulfate, lauric monoglyceride sodium sulfate, sodium lauryl sulfate, sodium laureth sulfate, potassium lauryl sulfate, potassium laureth sulfate, sodium trideceth sulfate, sodium tridecyl sulfate, ammonium trideceth sulfate, ammonium tridecyl sulfate, sodium cocoyl isethionate, disodium laureth sulfosuccinate, sodium methyl oleoyl taurate, sodium laureth carboxylate, sodium trideceth carboxylate, sodium-monoalkyl phosphates, sodium dialkyl phosphates, sodium lauryl sarcosinate, lauroyl sarcosine, cocoyl sarcosinate, ammonium cocyl sulfate, sodium cocyl sulfate, potassium cocyl sulfate, monoethanolamine cocyl sulfate, sodium tridecyl benzene sulfonate, sodium dodecyl benzene sulfonate, and branched anionic surfactants, such as sodium trideceth sulfate, sodium tridecyl sulfate, ammonium trideceth sulfate, and ammonium tridecyl sulfate.

The cation of any anionic surfactant is typically sodium but may alternatively be potassium, lithium, calcium, magnesium, ammonium, or an alkyl ammonium having up to 6 aliphatic carbon atoms including isopropylammonium, monoethanolammonium, diethanolammonium, and triethanolammonium. Ammonium and ethanolammonium salts are generally more soluble that the sodium salts. Mixtures of the above cations may be used.

The amount of anionic surfactant in the composition can be typically of from 5 to 30% by weight.

a3) Structurant

The surfactant system can comprise a structurant (that might be itself a surfactant or not). Examples of structurants include:

- alkanolamides, such as cocamide MEA, cocamide DEA, or cocamide MIPA,
- fatty acids, optionally insaturated et/or branched, preferably C$_8$-C$_{24}$ fatty acids, such as lauric acid, oleic acid, isostéarique acid,
- fatty acids esters optionally unsaturated and/or branched, preferably preference en C$_8$-C$_{24}$ fatty acids esters, such as methyl esters or for example lauric acid, oleic acid, isostéarique acid,

- alcoxylated, for example (poly)ethoxylated or (poly) propoxylated, fatty acids esters optionally unsaturated and/or branched, preferably preference en $C_8$-$C_{24}$ fatty acids esters, for example propylene glycol isostrearate,
- fatty alcohols, optionally ethoxylated and/or propoxylated, preferably with a low number of ethoxy and/or propoxyunits, preferably laureth-2, laureth-3, laureth-4
- trihydroxystearin.

Some of the above mentionned compounds might be condidered as non ionic surfactants.

The amount of structurant in the composition can be typically of from 1 to 10% by weight.

a4) amphoteric or zwitterionic surfactant

In some embodiments the surfactant system comprises an amphoteric or zwitterionic surfactant. Examples of amphoteric or zwitterionic surfactants that can be used include the following (INCI names):

Almondamidopropyl Betaine; Apricotamidopropyl Betaine; Avocadamidopropyl Betaine; Babassuamidopropyl Betaine; Behenamidopropyl Betaine; Behenyl Betaine; Behenyl Hydroxyethyl Imidazoline; Canolamidopropyl Betaine; Capryl/Capramidopropyl Betaine; Capryl Hydroxyethyl Imidazoline; Caprylyl Hydroxyethyl Imidazoline; Cetyl Betaine; Coco-Betaine; Cocamidoethyl Betaine; Cocamidopropyl Betaine; Cocamidopropyl Hydroxysultaine; Coco-Hydroxysultaine; Coco/Oleamidopropyl Betaine; Cupuassuamidopropyl Betaine; Coco-Sultaine; DEA-Cocoamphodipropionate; DEA-Lauraminopropionate; Decyl Betaine; Cocoyl Hydroxyethyl Imidazoline; Decyl Mercaptomethylimidazole; Disodium Caproamphodiacetate; Disodium Caproamphodipropionate; Disodium Capryloamphodiacetate; Disodium Capryloamphodipropionate; Disodium Cocaminopropyl Iminodiacetate; Disodium Cocoamphocarboxyethylhydroxypropylsulfonate; Disodium Cocoamphodiacetate; Disodium Cocoamphodipropionate; Disodium Dicarboxyethyl Cocopropylenediamine; Disodium Isostearoamphodiacetate; Disodium Isostearoamphodipropionate; Disodium Laureth-5 Carboxyamphodiacetate; Disodium Lauriminodiacetate; Disodium Lauriminodipropionate; Disodium Lauriminodipropionate Tocopheryl Phosphates; Disodium Lauroamphodiacetate; Disodium Lauroamphodipropionate; Disodium Oleoamphodipropionate; Disodium PPG-2-Isodeceth-7 Carboxyamphodiacetate; Disodium Steariminodipropionate; Disodium Stearoamphodiacetate; Disodium Tallowamphodiacetate; Disodium Tallowiminodipropionate; Disodium Wheatgermamphodiacetate; Glutamylamidoethyl Imidazole; Hydrogenated Tallow Betaine; Hydroxyethyl Carboxymethyl Cocamidopropylamine; Hydroxyethyl Diphenyl Imidazoline; Isostearamidopropyl Betaine; Isostearyl Hydroxyethyl Imidazoline; Lauramidobutyl Guanidine Acetate; Lauramidobutyl Guanidine HCl; Lauramidopropyl Betaine; Lauramidopropyl Hydroxysultaine; Lauryl Betaine; Lauryl Hydroxyethyl Imidazoline; Lauryl Hydroxysultaine; Methoxycinnamidopropyl Hydroxysultaine; Milkamidopropyl Betaine

Minkamidopropyl Betaine; Myristamidopropyl Betaine; Myristamidopropyl Hydroxysultaine; Myristyl Betaine Myristyl Hydroxyethyl Imidazoline; Oleamidopropyl Betaine; Oleamidopropyl Hydroxysultaine; Oleyl Betaine; Oleyl; Hydroxyethyl Imidazoline; Olivamidopropyl Betaine; Palmamidopropyl Betaine; Palmitamidopropyl Betaine; Palm Kernelamidopropyl Betaine; Ricinoleamidopropyl Betaine; Sesamidopropyl Betaine; Sodium C12-15 Alkoxypropyl Iminodipropionate; Sodium Caproamphoacetate; Sodium Caproamphohydroxypropylsulfonate; Sodium Caproamphopropionate; Sodium Capryloamphoacetate; Sodium Capryloamphohydroxypropylsulfonate; Sodium Capryloamphopropionate; Sodium Cocaminopropionate; Sodium Cocoabutteramphoacetate; Sodium Cocoamphoacetate; Sodium Cocoamphohydroxypropylsulfonate; Sodium Cocoamphopropionate; Sodium Cornamphopropionate; Sodium Dicarboxyethylcoco Phosphoethyl Imidazoline; Sodium Isostearoamphoacetate; Sodium Isostearoamphopropionate; Sodium Lauraminopropionate; Sodium Lauriminodipropionate; Sodium Lauroamphoacetate; Sodium Lauroamphohydroxypropylsulfonate; Sodium Lauroampho PG-Acetate Phosphate; Sodium Lauroamphopropionate; Sodium Myristoamphoacetate; Sodium Oleoamphoacetate; Sodium Oleoamphohydroxypropylsulfonate; Sodium Oleoamphopropionate; Sodium Olivamphoacetate; Sodium Palmamphoacetate; Sodium Peanutamphoacetate; Sodium Ricinoleoamphoacetate; Sodium Sesamphoacetate; Sodium Stearoamphoacetate; Sodium Stearoamphohydroxypropylsulfonate; Sodium Stearoamphopropionate; Sodium Sunflowerseedamphoacetate; Sodium Sweetalmondamphoacetate; Sodium Tallamphopropionate Sodium Tallowamphoacetate; Sodium Undecylenoamphoacetate; Sodium Undecylenoamphopropionate; Sodium Wheat Germamphoacetate; Soyamidopropyl Betaine; Soy Hydroxyethyl Imidazoline; Stearamidopropyl Betaine; Stearyl Betaine Stearyl Hydroxyethyl Imidazoline; TEA-Lauraminopropionate; TEA-Myristaminopropionate; Tall Oil Benzyl Hydroxyethyl Imidazolinium Chloride; Tall Oil Hydroxyethyl Imidazoline; Tallowamidopropyl Betaine; Tallowamidopropyl Hydroxysultaine; Tallow Betaine; Tallow Dihydroxyethyl Betaine; Tallow Hydroxyethyl Imidazoline; Undecylenamidopropyl Betaine; Wheat Germamidopropyl Betaine

Most preferred amphoteric or zwitterionic surfactant include betaines; such as alkylbetaïnes or alkylamidopropyl betaïnes, or imidazoline derivatives, such as alkylamphoacetates or alkylamphodiacetates, where the alkyl is a C8-C22 alkyl group or mixture or C8-C22 alkyl group, optionally comprising insaturations.

The amount of amphoteric or zwitterionic surfactant if present in the composition can be typically of from 0.1 to 10% by weight.

a5) electrolyte

In some embodiments the surfactant system comprises an electrolyte (electrolytes are considered as part of the surfactant system as they can assist in forming the structured form).

Suitable electrolytes include organic salts, inorganic salts, and mixtures thereof, as well as polyelectrolytes, such as uncapped polyacrylates, polymaleates, or polycarboxylates, lignin sulfonates or naphthalene sulfonate formaldehyde copolymers. The electrolyte typically comprises a salt having a cationic component and an anionic component. Suitable cations may be monovalent or multivalent, may be organic or inorganic, and include, for example, sodium, potassium, lithium, calcium, magnesium, cesium, and lithium cations, as well as mono-, di- tri- or quaternary ammonium or pyridinium cation. Suitable anions may be a monovalent or multivalent, may be organic or inorganic, and include, for example, chloride, sulfate, nitrate, nitrite, carbonate, citrate, cyanate acetate, benzoate, tartarate, oxalate, phosphate, and phosphonate anions. Suitable electrolytes include, for example, salts of multivalent anions with monovalent cations, such as potassium pyrophosphate, potassium tripolyphosphate, and sodium citrate, salts of multivalent cations with monovalent anions, such as calcium chloride, calcium bromide, zinc halides, barium chloride, and calcium nitrate, and salts of monovalent cations with monovalent anions, such as sodium chloride, potassium chloride, potassium iodide, sodium bromide, ammonium bromide, alkali metal nitrates, and ammonium nitrates. Electrolyte may be added as a separate component or in combination with other components of the composition of the present invention.

In one embodiment, the electrolyte comprises NaCl, $NH_4Cl$, or a mixture thereof. The use of $NH_4Cl$ as at least a portion of the electrolyte component of the composition of the present invention has been found to offer improved efficiency, that is, compared to other electrolytes, a smaller amount of $NH_4Cl$ is needed to, in combination with the other components of the composition, provide a structured surfactant composition having an opaque visual appearance and exhibiting a yield strength of greater than 0 Pascal. In one embodiment, the electrolyte comprises $NH_4Cl$.

The amount of electrolyte if present in the composition can be typically of from 0.1 to 10% by weight.

b oil

The composition comprises at least an oil. The oil can be for example:

b1) a silicone oil,
b2) an oil of mineral origin, or

- a mixture or association thereof.

The oil is typically present in the composition in the form of dispersed particles or droplets.

The composition can typically comprise from 0.05 to 10% by weight, preferably from 0.1 to 5%; of the oil.

b1) silicone oils

In one embodiment the composition comprises a silicone oil. Silicone oils are known by the one skilled in the art. These are often referred to as polyorganosiloxanes. In the present application the terms "silicone" or "polyorganosiloxane" can be used indifferently. The term "silicone" or "polyorganosiloxane" is understood to mean any organosiloxane compound comprising alkyl (for example methyl) groups and/or functionalized by groups other than alkyl groups. Silicones can be linear, cyclic, or branched polymers or oligomers of monomeric silicon/oxygen (organosiloxane) monomers, optionally bearing some further functional groups. The polymeric backbone is typically made up of alternating silicon and oxygen atoms. The silicon atoms may carry a wide variety of substituents which can be the same or different. Functional endblocking groups may carry nitrogen or hydroxyl moieties.

The polyorganosiloxane is advantageously (in shampoos and conditioners in particular) a nonvolatile and water-insoluble polyorganosiloxane. It advantageously exhibits a viscosity of between 1000 and 2 000 000 mPa·s, preferably between 5000 and 500 000 mPa·s. The polyorganosiloxane can in particular be a polydimethylorganosiloxane ("PDMS", INCI name: dimethicone), or a polyorganosiloxane exhibiting amine groups (for example, amodimethicone according to the INCI name), quaternary ammonium groups (for example, silicone quaternium-1 to - 10 according to the INCI name), hydroxyl groups (terminal or non terminal), polyoxyalkylene groups, for example polyethylene oxide and/or polypropylene oxide groups (as terminal groups, as blocks within a PDMS chain or as grafts), or several of these groups.

The amount of silicone oil present in the composition can typically be from 0.1% to 5% by weight, for example from 0.5% to 1.5% or 2% by weight.

The silicone oil (polyorganosiloxane) is preferably present in the composition in an emulsion form (liquid silicone droplets dispersed in the aqueous phase). The silicone oil can be present in the composition in the form of:

- a microemulsion with a particle size of lower than 0,15 $\mu$m,
- an emulsion with a particle size of from 0,15 $\mu$m to lower than 1 $\mu$m, or of from 1 $\mu$m to lower than 1.5 $\mu$m or of from 1.5 $\mu$m to lower than 2 $\mu$m, or from 2 $\mu$m to lower than 2.5 $\mu$m, or from 2.5 $\mu$m to lower than 4 $\mu$m, or from 4 $\mu$m to lower than 10 $\mu$m, or from 10 $\mu$m to lower than 30 $\mu$m, or from 30 $\mu$m to 100 $\mu$m. Sizes herein refer to mean sizes of the droplets.

The droplets of the emulsion can be more or less large in size. Reference may thus be made to microemulsions, to miniemulsions or to macroemulsions. In the present patent application, the term "emulsion" covers in particular all these types of emulsion. Without wishing to be committed to any one theory, it is specified that microemulsions are generally thermodynamically stable systems generally comprising large amounts of emulsifying agents. The other emulsions are generally systems in the non-thermodynamically stable state which retain for a certain time, in the metastable state, the mechanical energy provided during the emulsification. These systems generally comprise lesser amounts of emulsifying agents.

The emulsions can be obtained by mixing the carrier, preferably aqueous carrier, the polyorganosiloxane and generally an emulsifying agent, and then emulsifying. It is possible to speak of in situ emulsification.

The compositions in the emulsion form can also be obtained by mixing the carrier, preferably aqueous carrier, with a pre-prepared emulsion of droplets comprising the polyorganosiloxane in an external phase which is preferably miscible with the cosmetically acceptable carrier, preferably of the same nature as said carrier, preferably an aqueous carrier. This embodiment may be preferred as it is simple to implement. In addition, this embodiment is particularly suitable for the implementation of cosmetic compositions in which the polyorganosiloxane is in the microemulsion form. It is possible to speak of pre-emulsification.

According to a specific embodiment, the emulsion is a microemulsion, the size of the droplets of which is less than 0.15 $\mu$m. In this embodiment, the composition preferably comprises a proportion of emulsifying agent of greater than 10% by weight, preferably at least 15% by weight, with respect to the weight of polyorganosiloxane.

The size of the microemulsion droplets can be measured on an emulsion prepared prior to this introduction into the cosmetic composition by dynamic light scattering (QELS), for example as described below. The equipment used is, for example, composed of a Spectra-Physics 2020 laser, of a Brookhaven 2030 correlator and of the associated computing. As the sample is concentrated, it is diluted in deionized water and filtered through a 0.22 $\mu$m filter in order, at the end, to be at 2% by weight. The diameter obtained is an apparent diameter. The measurements are carried out at angles of 90° and 135°. For the size measurements, in addition to the conventional analysis by cumulants, the autocorrelation function is run in three ways (the exponential sampling or EXPSAM described by Pr. Pike, the "Non Negatively Constrained Least Squares" or NNLS method and the CONTIN method described by Pr. Provencher) which each give a size distribution weighted by the scattered intensity and not by the weight or the number. The refractive index and the viscosity of the water are taken into account.

According to an advantageous form, the microemulsion is transparent. The microemulsion can, for example, exhibit a transmittance of at least 90%, preferably of at least 95%, at a wavelength of 600 nm, measured, for example, using a Lambda 40 UV-Vis spectrometer at a concentration of 0.5% by weight in water. In this context, the cosmetic composition can advantageously be transparent. It can, for example, exhibit a transmittance of at least 90%, preferably of at least 95%, at a wavelength of 600 nm, measured, for example, using a Lambda 40 UV-Vis spectrometer.

According to another specific embodiment, the emulsion is an emulsion for which the mean size of the droplets is greater than or equal to 0.15 $\mu$m, for example greater than 0.5 $\mu$m, or than 1 $\mu$m, or than 2 $\mu$m, or than 10 $\mu$m, or than 20 $\mu$m, and preferably less than 100 $\mu$m. The size of the droplets can be measured, by optical microscopy and/or laser particle sizing (Horiba LA-910 laser scattering analyzer), on an emulsion prepared prior to its introduction into the cosmetic composition or directly on the cosmetic composition diluted in water. In this embodiment, the composition preferably comprises a proportion of emulsifying agent of less than 10% by weight, with respect to the weight of polyorganosiloxane.

Emulsifying agents of use in the preparation of polyorganosiloxane emulsions are in particular nonionic surfactants, preferably polyalkoxylated surfactants, for example chosen from alkoxylated fatty alcohols, alkoxylated triglycerides, alkoxylated fatty alcohols, alkoxylated sorbitan esters, alkoxylated fatty amines, alkoxylated di(1-phenylethyl)phenols, alkoxylated tri(1-phenylethyl)phenols and alkoxylated alkylphenols, where the number of alkoxy units, more particularly oxyethylene and/or oxypropylene units, is such that the HLB value is greater than or equal to 10.

Mention may be made, among the silicone derivatives which are soluble in the water of the composition, inter alia, of dimethicone copolyols (Mirasil DMCO, sold by Rhodia Chimie).

As relates to the silicones which are provided in the form of dispersions which are insoluble in the water of the composition, use may suitably be made of water-insoluble and nonvolatile polyorganosiloxanes, among which may be mentioned polyalkylsiloxane, polyarylsiloxane or polyalkylarylsiloxane oils, gums or resins or their water-insoluble functionalized derivatives, or their mixtures, which are nonvolatile.

Said organopolyosiloxanes are regarded as water-insoluble and nonvolatile if their solubility in water is less than 50 g/liter and their intrinsic viscosity is at least 3000 mPa·s at 25°C.

Mention may be made, as examples of water-insoluble and nonvolatile polyorganosiloxanes or silicones, of silicone gums, such as, for example, the diphenyl dimethicone gum sold by Rhodia, and preferably the polydimethylsiloxanes exhibiting a viscosity at least equal to $6 \times 10^5$ mPa·s at 25°C and more preferably still those with a viscosity of greater than $2 \times 10^6$ mPa·s at 25°C, such as Mirasil DM 500 000®, sold by Rhodia.

According to the invention, the water-insoluble and nonvolatile polyorganosiloxane or silicone occurs in a form dispersed within the cosmetic composition including it.

The water-insoluble and nonvolatile polyorganosiloxane or silicone exists in the form of particles or droplets, the size of which can be chosen according to the nature of the cosmetic composition or the performance desired for said composition. Generally, this size can vary from 0.01 to 70 microns.

In order to facilitate the use thereof, these polyorganosiloxanes can be dispersed or dissolved beforehand in volatile or nonvolatile silicone derivatives of low viscosity and then emulsified in the cosmetic composition.

Mention may be made, among these silicones of low viscosity, of volatile cyclic silicones and polydimethylsiloxanes of low weight.

Use can also be made of functionalized silicone derivatives, such as aminated derivatives, directly in the form of emulsions or starting from a preformed microemulsion. They can be compounds known under the term of aminated silicones or hydroxylated silicones. Mention is made of Mirasil ADM-E (amodimethicone), sold by Rhodia, and dimethiconol.

Mention is in particular made, as polyorganosiloxanes which can be used, of:

- polyorganosiloxanes comprising -Si(CH₃)₂O- units and -SiY(CH₃)O- units where Y is a -(CH₂)₃-NH(CH₂)₂-NH₂ or-(CH₂)₃-NH₂ group,
- polyorganosiloxanes comprising -Si(CH₃)₂O- units and HO-Si(CH₃)₂O- terminal units and/or-Si(CH₃)(OH)O- nonterminal units,
- polyorganosiloxanes comprising -Si(CH₃)₂O- units and -SiY(CH₃)O- units where Y is -L$^X$-Z$^x$-Palc where L$^X$ is a divalent connecting group, preferably an alkylene group, Z$^X$ is a covalent bond or a divalent joining group comprising a heteroatom, Palc is a group of formula [OE]$_s$-[OP]$_t$-X', in which OE is a group of formula -CH₂-CH₂-O-, OP is a group of formula -CH₂-CHCH₃-O- or -CHCH₃-CH₂-O-, X' is a hydrogen atom or a hydrocarbon group, s is a mean number greater than 1 and t is a mean number greater than or equal to 0,
- polyorganosiloxanes, the chain of which comprises at least one block comprising units of formula -Si(CH₃)₂O- units and at least one -[OE]$_s$-[OP]$_t$- block,
- polyorganosiloxanes comprising -Si(CH₃)₂O- units and/or -Si(CH₃)RO- and/or-SiR₂O- and/or R-Si(CH₃)₂O- and/or H₃C-SiR₂O- and/or R-SiR₂O- units, where R, which can be identical or different, is an alkyl group other than a methyl group, an aryl group, an alkylaryl group or an aralkyl group.

Examples of silicone oils that can be used include the following (INCI names): Amino Bispropyl Dimethicone; Aminopropyl Dimethicone; Aminopropyl Phenyl Trimethicone; Amodimethicone; Amodimethicone Hydroxystearate; Amodimethicone/Silsesquioxane Copolymer; Behentrimonium Dimethicone PEG-8 Phthalate; Bisamino PEG/PPG-41/3 Aminoethyl PG-Propyl Dimethicone; Bis-Aminopropyl Dimethicone; Bis-Aminopropyl/Ethoxy Aminopropyl Dimethicone; Bis-Butyldimethicone Polyglyceryl-3; Bis-Butyloxyamodimethicone/PEG-60 Copolymer; Bis(C13-15 Alkoxy) Hydroxybutamidoamodimethicone; Bis(C13-15 Alkoxy) PG-Amodimethicone; Bis-Hydroxyethoxypropyl Dimethicone Beeswax Esters; Bis-Hydroxyethoxypropyl Dimethicone Isostearate; Bis-Isobutyl PEG-14/Amodimethicone Copolymer; Bis-Isobutyl PEG-15/Amodimethicone Copolymer; Bis-PEG-1 Dimethicone; Bis-PEG-4 Dimethicone; Bis-PEG-8 Dimethicone; Bis-PEG-12 Dimethicone Bis-PEG-20 Dimethicone; Bis-PEG-12 Dimethicone Beeswax; Bis-PEG-12 Dimethicone Candelillate; Bis-PEG-10 Dimethicone/Dimer Dilinoleate Copolymer; Bis-PEG-15 Methyl Ether Dimethicone; Bisphenylhexamethicone; Bis-Phenylpropyl Dimethicone; Bis-(Polyglyceryl-3 Oxyphenylpropyl) Dimethicone; Bis(PPG-7 Undeceneth-21) Dimethicone; Borage Seed Oil PEG-7 Dimethicone Esters; C30-45 Alkyl Cetearyl Dimethicone Crosspolymer; C26-28 Alkyl Dimethicone; Cetearyl Dimethicone/Vinyl Dimethicone Crosspolymer; Cetrimonium Carboxydecyl PEG-8 Dimethicone; Cetyl Triethylmonium Dimethicone PEG-8 Phthalate; Cetyl Triethylmonium Dimethicone PEG-8 Succinate; Cyclohexasiloxane; Cyclomethicone; Cyclopentasiloxane; Cyclophenylmethicone; Cyclotetrasiloxane; Cyclotrisiloxane; DEA PG-Propyl PEG/PPG-18/21 Dimethicone; Dilinoleamidopropyl Dimethylamine, Dimethicone; Dimethicone PEG-7 Phosphate; Dimethicone Hydroxypropyl Trimonium Chloride; Dimethicone/Mercaptopropyl Methicone Copolymer; Dimethicone PEG-15 Acetate; Dimethicone PEG-8 Adipate; Dimethicone PEG-7 Avocadoate; Dimethicone PEG-8 Avocadoate; Dimethicone PEG-8 Beeswax; Dimethicone PEG-8 Borageate; Dimethicone PEG-7 Cocoate; Dimethicone PEG-7 Isostearate; Dimethicone PEG-7 Lactate; Dimethicone PEG-8 Lanolate; Dimethicone PEG-8 Meadowfoamate; Dimethicone PEG-7 Olivate; Dimethicone PEG-8 Olivate; Dimethicone PEG-8 Phosphate; Divinyldimethicone/Dimethicone Copolymer; Dimethicone PEG-7 Phthalate; Dimethicone PEG-8 Phthalate; Dimethicone PEG-7 Succinate; Dimethicone PEG-8 Succinate; Dimethicone PEG-7 Sulfate; Dimethicone PEG-7 Undecylenate; Dimethicone Propyl PG-Betaine; Dimethicone/Silsesquioxane Copolymer; Dimethiconol Arginine; Dimethiconol Cysteine; Dimethiconol Lactate; Dimethiconol Methionine; Dimethiconol Panthenol; Dimethiconol/Silsesquioxane Copolymer; Di-Methoxycinnamidopropyl Ethyldimonium Chloride Ether; Dimethoxysilyl Ethylenediaminopropyl Dimethicone; Dimethylaminopropylamido PCA Dimethicone; Diphenyl Amodimethicone; Diphenylisopropyl Dimethicone; Diphenylsiloxy Phenyl Trimethicone; Glycidoxy Dimethicone; Hexyl Dimethicone; Hydrolyzed Collagen PG-Propyl Dimethiconol; Hydrolyzed Collagen PG-Propyl Methylsilanediol; Hydrolyzed Collagen PG-Propyl Silanetriol; Hydrolyzed Keratin PG-Propyl Methylsilanediol; Hydrolyzed Sesame Protein PG-Propyl Methylsilanediol; Hydrolyzed Silk PG-Propyl Methylsilanediol; Hydrolyzed Silk PG-Propyl Methylsilanediol Crosspolymer; Hydrolyzed Soy Protein/Dimethicone PEG-7 Acetate; Hydrolyzed Soy Protein PG-Propyl Methylsilanediol; Hydrolyzed Vegetable Protein PG-Propyl Silanetriol; Hydrolyzed Wheat Protein/Cystine Bis-PG-Propyl Silanetriol Copolymer; Hydrolyzed Wheat Protein PG-Propyl Methylsilanediol; Hydrolyzed Wheat Protein PG-Propyl Silanetriol; Hydroxypropyldimethicone; Isopolyglyceryl-3 Dimethicone; Isopolyglyceryl-3 Dimethiconol; Lauryl PEG-9 Polydimethylsiloxyethyl Dimethicone; Lauryl Polyglyceryl-3 Polydimethylsiloxyethyl Dimethicone; Linoleamidopropyl PG-Dimonium Chloride Phosphate Dimethicone; Methoxy Amodimethicone/Silsesquioxane Copolymer; Methyleugenyl PEG-8 Dimethicone; Methylsilanol Acetylmethionate; Methylsilanol Elastinate; Methyl Trimethicone; Nylon-611/Dimethicone Copolymer; PCA Dimethicone; PEG-8 Amodimethicone; PEG-3 Dimethicone; PEG-8 Dimethicone; PEG-9 Dimethicone; PEG-10 Dimethicone; PEG-12 Dimethicone; PEG-14 Dimethicone; PEG-17 Dimethicone ; PEG-8 Distearmonium Chloride PG-Dimethicone; PEG-8 Methicone

PEG-6 Methicone Acetate; PEG-6 Methyl Ether Dimethicone; PEG-7 Methyl Ether Dimethicone; PEG-8 Methyl Ether Dimethicone; PEG-9 Methyl Ether Dimethicone; PEG-10 Methyl Ether Dimethicone; PEG-11 Methyl Ether Dimethicone; PEG-32 Methyl Ether Dimethicone ; PEG-10 Nonafluorohexyl Dimethicone Copolymer; PEG-12 Methyl Ether Lauroxy PEG-5 Amidopropyl Dimethicone; PEG-8 PG-Coco-Glucoside Dimethicone; PEG/PPG-28/21 Acetate Dimethicone; PEG/PPG-20/22 Butyl Ether Dimethicone; PEG/PPG-22/22 Butyl Ether Dimethicone; PEG/PPG-23/23 Butyl Ether Dimethicone; PEG/PPG-24/18 Butyl Ether Dimethicone; PEG/PPG-27/9 Butyl Ether Dimethicone; PEG/PPG-10/2 Dimethicone; PEG/PPG-20/23 Dimethicone; PEG/PPG-20/22 Methyl Ether Dimethicone; PEG/PPG-24/24 Methyl Ether Glycidoxy Dimethicone; PEG/PPG-10/3 Oleyl Ether Dimethicone; PEG-4 Trifluoropropyl Dimethicone Copolymer; PEG-8 Trifluoropropyl Dimethicone Copolymer; PEG-10 Trifluoropropyl Dimethicone Copolymer; PG-Amodimethicone; Phenyl Methiconol;

Phenylpropyldimethylsiloxysilicate; Phenylpropyl Ethyl Methicone; Phenyl Propyl Trimethicone; Phenyl Trimethicone; Polydimethylsiloxy PPG-13 Butyl Ether Silsesquioxane; Polyglyceryl-3 Disiloxane Dimethicone; Polyglyceryl-3 Polydimethylsiloxyethyl Dimethicone; Polysilicone-1; Polysilicone-2; Polysilicone-3; Polysilicone-4; Polysilicone-5; Polysilicone-6; Polysilicone-7; Polysilicone-8; Polysilicone-10; Polysilicone-13; Polysilicone-14; Polysilicone-18; Polysilicone-18 Cetyl Phosphate; Polysilicone-18 Stearate; PPG-12 Butyl Ether Dimethicone; PPG-12 Dimethicone; PPG-27 Dimethicone; Propoxytetramethyl Piperidinyl Dimethicone; Quaternium-80; Silicone Quaternium-1; Silicone Quaternium-2; Silicone Quaternium-2 Panthenol Succinate; Silicone Quaternium-3; Silicone Quaternium-4; Silicone Quaternium-5; Silicone Quaternium-6; Silicone Quaternium-7; Silicone Quaternium-8; Silicone Quaternium-9; Silicone Quaternium-10; Silicone Quaternium-11; Silicone Quaternium-12; Silicone Quaternium-15; Silicone Quaternium-16; Silicone Quaternium-16/Glycidoxy Dimethicone Crosspolymer; Silicone Quaternium-17; Silicone Quaternium-18; Silicone Quaternium-20; Sodium Dimethicone PEG-7 Acetyl Methyltaurate; Stearalkonium Dimethicone PEG-8 Phthalate; Steardimonium Hydroxypropyl Panthenyl PEG-7 Dimethicone Phosphate Chloride; Steardimonium Hydroxypropyl PEG-7 Dimethicone Phosphate Chloride; Trideceth-9 PG-Amodimethicone; Trifluoropropyl Cyclopentasiloxane; Trifluoropropyl Cyclotetrasiloxane; Trifluoropropyl Dimethicone; Trimethylsiloxyamodimethicone; Trimethylsiloxyphenyl Dimethicone; Gluconamidopropyl Aminopropyl Dimethicone; Cetrimonium Dimethicone PEG-7 Phthalate; Stearyl Aminopropyl Methicone; Myristylamidopropyl Dimethylamine Dimethicone PEG-7 Phosphate; Potassium Dimethicone PEG-7 Panthenyl Phosphate; Sodium PG-Propyldimethicone Thiosulfate Copolymer; Sodium PG-Propyl Thiosulfate Dimethicone; Tetrabutoxypropyl Trisiloxane.

In some preferred embodiements, the silicone oil is:

- a dimethicone
- an amodimethicone
- a dimethiconol,
- a PEG-dimethicone, or
- a mixtutre or association thereof.

The composition can typically comprise from from 0.1 to 5% by weight, for example from 0,2 to 1.5% or from 1.5 to 3.5%, of the silocone oil.

b2) an oil of mineral origin

In one embodiment the oil is an oil of mineral origin. Such compounds are known by the one skilled in the art. Examples of oils of mineral origin that can be used include the following (INCI names):

- Petrolatum,
- Mineral Oil,
- Hydrogenated Polydodecene,
- Hydrogenated Polydecene,
- Polydecene.

c) cationic or ampholytic polymer

In one embodiment the composition comprises at least one cationic or ampholytic copolymer. The composition can comprise a mixture or association of several cationic or ampholytic polymers. Such compounds can assist in oil deposition. They might also provide some conditioning effects. In some embodiments these compounds can also help in increasing the viscosity and/or the stability of the composition. They can for example enhance the appearance and feel of hair, increase hair body or suppleness, facilitate styling, improve gloss or sheen and improve the texture of hair that has been damaged by chemical or physical action. They can provide anti-static effect, in altering the static electrical properties of hair.

The at least one cationic or ampholytic polymer can be for example c1) a modified polysaccharide, for example a cationic cellulose or a cationic guar, c2) a synthetic cationic polymer, for example polymers comprising units having a quaternary ammonium group or a tertiary ammonium group, and optionally neutral units c3) a synthetic ampholytic copolymer, for example polymers comprising units having a quaternary ammonium group or a tertiary ammonium group, units having an anionic (usually acidic) group and optionally neutral units, or
a mixture or association thereof.

Such compounds are known by the one skilled in the art. Examples of useful compounds include (INCI names):

Polyquaternium-1; Polyquaternium-2; Polyquaternium-4; Polyquaternium-5; Polyquaternium-6; Polyquaternium-7; Polyquaternium-8; Polyquaternium-9; Polyquaternium-10; Polyquaternium-11; Polyquaternium-12; Polyquaternium-13; Polyquaternium-14; Polyquaternium-15; Polyquaternium-16; Polyquaternium-17; Polyquaternium-18; Polyquaternium-19; Polyquaternium-20; Polyquaternium-22; Polyquaternium-24; Polyquaternium-27; Polyquaternium-28; Polyquaternium-29; Polyquaternium-30; Polyquaternium-31; Polyquaternium-32; Polyquaternium-33; Polyquaternium-34 Polyquaternium-35; Polyquaternium-36; Polyquaternium-37; Polyquaternium-39; Polyquaternium-43; Polyquaternium-44; Polyquaternium-45; Polyquaternium-46; Polyquaternium-47; Polyquaternium-48; Polyquaternium-49; Polyquaternium-50; Polyquaternium-52; Polyquaternium-53; Polyquaternium-54; Polyquaternium-55; Polyquaternium-56; Polyquaternium-57; Polyquaternium-58; Polyquaternium-59; Polyquaternium-60; Polyquaternium-63; Polyquaternium-64; Polyquaternium-65; Polyquaternium-66; Polyquaternium-67; Polyquaternium-70; Polyquaternium-73; Polyquaternium-74; Polyquaternium-75; Polyquaternium-76 ; Polyquaternium-85; Polyquaternium-86; Polybeta-Alanine; Polyepsilon-Lysine; Polylysine; PEG-8/SMDI Copolymer; PPG-12/SMDI Copolymer; PPG-51/SMDI Copolymer; PPG-7/Succinic Acid Copolymer; IPDI/PEG-15 Cocamine Copolymer; IPDI/PEG-15 Cocamine Copolymer Dimer Dilinoleate; IPDI/PEG-15 Soyamine Copolymer; IPDI/PEG-15 Soyamine Oxide Copolymer; IPDI/PEG-15 Soyethonium Ethosulfate Copolymer; Polyquaternium-4/Hydroxypropyl Starch Copolymer; Cassia Hydroxypropyltrimonium Chloride; Chitosan Hydroxypropyltrimonium Chloride; Dextran Hydroxypropyltrimonium Chloride; Galactoarabinan Hydroxypropyltrimonium Chloride; Ginseng Hydroxypropyltrimonium Chloride; Guar Hydroxypropyltrimonium Chloride; Hydroxypropyl Guar Hydroxypropyltrimonium Chloride; Locust Bean Hydroxypropyltrimonium Chloride; Starch Hydroxypropyltrimonium Chlorid; Hydroxypropyltrimonium Hydrolyzed Wheat Starch; Hydroxypropyltrimonium Hydrolyzed Corn Starch; Hydroxypropyl Oxidized Starch PG-Trimonium Chloride; Tamarindus Indica Hydroxypropyltrimonium Chloride; Polyacrylamidopropyltrimonium Chloride; Polymethacrylamidopropyltrimonium Chloride; Polymethacrylamidopropyltrimonium Methosulfate; Propyltrimoniumchloride Methacrylamide/Dimethylacrylamide Copolymer; Acrylamide/Ethalkonium Chloride Acrylate Copolymer; Acrylamide/Ethyltrimonium Chloride Acrylate/Ethalkonium Chloride Acrylate Copolymer; Acrylates/Carbamate Copolymer; Adipic Acid/Methyl DEA Crosspolymer; Diethylene Glycol/DMAP Acrylamide/PEG-180/HDI Copolymer; Dihydroxyethyl Tallowamine/IPDI Copolymer; Dimethylamine/Ethylenediamine/Epichlorohydrin Copolymer; HEMA Glucoside/Ethylmethacrylate Trimonium Chloride Copolymer; Hydrolyzed Wheat Protein/PEG-20 Acetate Copolymer; Hydrolyzed Wheat Protein/PVP Crosspolymer; Ethyltrimonium Chloride Methacrylate/Hydroxyethylacrylamide Copolymer.

The composition can typically comprise from from 0.05 to 5% by weight, for preferably from 0.1 to 1.5%, for example from 0.1 to 0.4% or from 0.4 to 1%, of the cationic or ampholytic polymer.

d) other ingredients

The composition is an aqueous composition. Thus it comprises water. All or a part of water can be introduced as part of the products used to make the composition (if these products comprise some water, the active % being thus of less than 100%). All or a part of water can be introduced independently, at various stage, in amounts such the composition is completed to 100%.

The composition might comprise some further ingredients. Further ingredients can impart some further specific properties to the composition. The one skilled in the art knows such ingredients and/or properties that can be associated to these and/or appropriate amounts.

In one embodiment the composition comprises a rheoloqy modifier. These are known by the one skilled in the art. Rheology modifiers can be used to adjust the viscosity and/or the stability of the composition. Some useful and popular rheology modifiers are for example polyacrylates polymers (including copolymers). Some useful rheology modifiers are mineral such as clays. Some useful and popular rheology modifiers are natural gums or chemical derivatives thereof.

It is mentioned that some compounds might have rheology modifying effect while being cationic or amphoteric polymers.

Examples of rheology modifiers of natural origin, optionally chemically modified, that can be used include (INCI names):

Agar; Agarose; Alcaligenes Polysaccharides; Algin; Alginic Acid; Ammonium Alginate; Amylopectin; Arachis Hypogaea (Peanut) Flour; Ascorbyl Methylsilanol Pectinate; Astragalus Gummifer Gum; Attapulgite; Avena Sativa (Oat) Kernel Flour; Butoxy Chitosan; Bentonite; Biotite; Caesalpinia Spinosa Gum; Calcium Alginate; Calcium Carboxymethyl Cellulose; Calcium Carrageenan; Calcium Lignosulfonate; Calcium Starch Octenylsuccinate; C12-16 Alkyl PEG-2 Hydroxypropyl Hydroxyethyl Ethylcellulose; Carboxybutyl Chitosan; Carboxymethyl Cellulose Acetate Butyrate; Carboxymethyl Chitin; Carboxymethyl Chitosan; Carboxymethyl Dextran; Carboxymethyl Hydroxyethylcellulose; Carboxymethyl Hydroxypropyl Guar; Carnitine; Cassia Gum; Cellulose Acetate Propionate Carboxylate; Cellulose Gum; Ceratonia Siliqua Gum; Cetyl Hydroxyethylcellulose; Chitosan Lauramide Succinamide; Cobalt DNA; Corallina Officinalis Powder; Croscarmellose; Cyamopsis Tetragonoloba (Guar) Gum; Dehydroxanthan Gum; Dextrin; Dihydrogenated Tallow Benzylmonium Hectorite; Disteardimonium Hectorite; Distarch Phosphate Acetate; Gelatin; Gellan Gum; Glyceryl Alginate; Glycine Soja (Soybean) Flour; Grateloupia Livida Powder; Guar Hydroxypropyltrimonium Chloride; Hectorite; Helianthus Annuus (Sunflower) Seed Flour; Hydrogenated Isocetyl Olivate; Hydrogenated Lecithin; Hydrogenated Potato Starch; Hydrolyzed Corn Starch Hydroxyethyl Ether; Hydroxybutyl Methylcellulose; Hydroxyethylcellulose; Hydroxyethyl Chitosan; Hydroxyethyl Ethylcellulose; Hydroxypropylcellulose; Hydroxypropyl Chitosan; Hydroxypropyl Guar; Hydroxypropyl Methylcellulose; Hydroxypropyl Methylcellulose Stearoxy Ether; Hydroxypropyl Starch; Hydroxypropyl Starch Phosphate; Hydroxypropyltrimonium Maltodextrin Crosspolymer; Hydroxypropyl Xanthan Gum; Locust Bean Gum; Levan; Linum Usitatissimum (Linseed) Seed Flour; Magnesium Alginate; Maltodextrin; Methylcellulose; Methyl Ethylcellulose; Methyl Hydroxyethylcellulose; Microcrystalline Cellulose; Natto Gum; Nitrocellulose; Nonoxynyl Hydroxyethylcellulose; Pectin; Perlite; Polyvinyl Alcohol-Potassium Alginate; Potassium Carrageenan; Potato Starch Modified; Propylene Glycol Alginate; Quaternium-18/Benzalkonium Bentonite; Quaternium-18 Hectorite; Quaternium-90 Bentonite; Rhizobian Gum; Sclerotium Gum; Sodium Arachidate; Sodium Carboxymethyl Chitin; Sodium Carboxymethyl Dextran; Sodium Carboxymethyl Beta-Glucan; Sodium Carboxymethyl Starch; Sodium Carrageenan; Sodium Cellulose Sulfate; Sodium Cyclodextrin Sulfate; Sodium Dextran Sulfate; Sodium Dimaltodextrin Phosphate; Sodium Hydroxypropyl Starch Phosphate; Sodium Isooctylene/MA Copolymer; Sodium Polygamma-Glutamate; Sodium Stearoxy PG-Hydroxyethylcellulose Sulfonate; Solanum Tuberosum (Potato) Starch; Starch Acetate/Adipate; Starch/Acrylates/Acrylamide Copolymer; Starch Hydroxypropyltrimonium Chloride; Stearalkonium Bentonite; Stearalkonium Hectorite; Sterculia Urens Gum; Sucrose Benzoate/Sucrose Acetate Isobutyrate/Butyl Benzyl Phthalate Copolymer; Tamarindus Indica Seed Gum; Tapioca Starch; Tara Gum; TEA-Alginate; Triticum Vulgare (Wheat) Starch; Welan Gum; Xanthan Gum; Yeast Beta-Glucan; Yeast Polysaccharides.

Examples of synthetic rheology modifyiers that can be used include (INCI names): Acrylamide/Ethalkonium Chloride Acrylate Copolymer; Acrylamide/Ethyltrimonium Chloride Acrylate/Ethalkonium Chloride Acrylate Copolymer;

Acrylamides Copolymer; Acrylamide/Sodium Acrylate Copolymer; Acrylamide/Sodium Acryloyldimethyltaurate Copolymer; Acrylates/Acetoacetoxyethyl Methacrylate Copolymer; Acrylates/Beheneth-25 Methacrylate Copolymer; Acrylates/C10-30 Alkyl Acrylate Crosspolymer; Acrylates/Ceteth-20 Itaconate Copolymer; Acrylates/Ceteth-20 Methacrylate Copolymer; Acrylates/Laureth-25 Methacrylate Copolymer; Acrylates/Palmeth-25 Acrylate Copolymer; Acrylates/Palmeth-25 Itaconate Copolymer; Acrylates/Steareth-50 Acrylate Copolymer; Acrylates/Steareth-20 Itaconate Copolymer; Acrylates/Steareth-20 Methacrylate Copolymer; Acrylates/Stearyl Methacrylate Copolymer; Acrylates/Vinyl Isodecanoate Crosspolymer; Acrylates/Vinyl Neodecanoate Crosspolymer; Acrylates Copolymer; Acrylates/Methoxy PEG-15 Methacrylate Copolymer; Acrylates/Steareth-20 Methacrylate Crosspolymer; Acrylates/Vinyl Isodecanoate Crosspolymer; Acrylates/VP Copolymer; Acrylic Acid/Acrylamidomethyl Propane Sulfonic Acid Copolymer; Acrylic Acid/Acrylonitrogens Copolymer; Acrylic Acid/Stearyl Methacrylate/Dimethicone Methacrylate Copolymer; Acrylic Acid/VP Crosspolymer; Adipic Acid/Methyl DEA Crosspolymer; Ammonium Acrylates/Acrylonitrogens Copolymer; Ammonium Acrylates Copolymer; Ammonium Acryloyldimethyltaurate/Steareth-8 Methacrylate Copolymer; Ammonium Acryloyldimethyltaurate/Vinyl Formamide Copolymer; Ammonium Acryloyldimethyltaurate/VP Copolymer; Ammonium Polyacryloyldimethyl Taurate; Ammonium Styrene/Acrylates Copolymer; Ammonium VA/Acrylates Copolymer; Bis-Butyldimethicone Polyglyceryl-3; Bis-Decyltetradecanyl IPDI/PEG-795 Copolymer; Bis-Stearyl IPDI/PEG-795 Copolymer; C4-24 Alkyl Dimethicone/Divinyldimethicone Crosspolymer; C6-14 Perfluoroalkylethyl Acrylate/HEMA Copolymer; Calcium Potassium Carbomer; Carbomer; Corn Starch/Acrylamide/Sodium Acrylate Copolymer; Decyltetradeceth-200 Behenate; Decyltetradeceth-200 Isostearate; Diallyloxyneohexyl Zirconium Tridecanoate; Dimethicone Crosspolymer; Dimethicone/PEG-10 Crosspolymer; Dimethicone/PEG-15 Crosspolymer ; Dimethiconol/Stearyl Methicone/Phenyl Trimethicone Copolymer; Dimethylol Urea/Phenol/Sodium Phenolsulfonate Copolymer; Dipentaerythrityl Pentaisostearate; Disodium Methylene Dinaphthalenesulfonate; Ditrimethylolpropane Isostearate/Sebacate; Ditrimethylolpropane Triethylhexanoate; Dimethicone Propyl PG-Betaine; Dimethylacrylamide/Acrylic Acid/Polystyrene ; Dimethylacrylamide/Sodium Acryloyldimethyltaurate Crosspolymer; Disteareth-75 IPDI; Disteareth-100 IPDI; DMAPA Acrylates/Acrylic Acid/Acrylonitrogens Copolymer; Erythrityl Triethylhexanoate; Ethyl Methacrylate Copolymer; Ethylene/MA Copolymer; Ethylene/Sodium Acrylate Copolymer; Ethylene/VA Copolymer; Ethylhexyl Hydroxystearoyl Hydroxystearate; Ethyl Trisiloxane; Glass; Glass Beads; Glyceryl Acrylate/Acrylic Acid Copolymer; Hydroxyethyl Acrylate/Sodium Acryloyldimethyl Taurate Copolymer; Hydroxyethyl PEI-1000; Hydroxyethyl PEI-1500; Hydroxypropyl Ethylenediamine Carbomer; Isobutylene/MA Copolymer; Isobutylene/Sodium Maleate Copolymer; Isopolyglyceryl-3 Dimethicone; Isopolyglyceryl-3 Dimethiconol; Isopropyl Ester of PVM/MA Copolymer; Lauryl PEG-9 Polydimethylsiloxyethyl Dimethicone; Lauryl Polyglyceryl-3 Polydimethylsiloxyethyl Dimethicone; Methacryloyl Ethyl Betaine/Acrylates Copolymer; Methoxy PEG-17/Dodecyl Glycol Copolymer; Methoxy PEG-22/Dodecyl Glycol Copolymer; Methoxy PEG-114/Polyepsilon Caprolactone; Octadecene/MA Copolymer; PEG-800; PEG-Crosspolymer; PEG-150/Decyl Alcohol/SMDI Copolymer; PEG-175 Diisostearate; PEG-150 Distearate; PEG-190 Distearate; PEG-15 Glyceryl Tristearate; PEG-140 Glyceryl Tristearate; PEG-240/HDI Copolymer Bis-Decyltetradeceth-20 Ether; PEG-200 Hydrgenated Glyceryl Palmate; PEG-100/IPDI Copolymer; PEG-180/Laureth-50/TMMG Copolymer; PEG-10/Lauryl Dimethicone Crosspolymer; PEG-15/Lauryl Dimethicone Crosspolymer; PEG-2M; PEG-5M; PEG-7M; PEG-9M; PEG-14M; PEG-20M; PEG-23M; PEG-25M; PEG-45M; PEG-65M; PEG-90M; PEG-115M; PEG-160M; PEG-180M ; PEG-120 Methyl Glucose Trioleate; PEG-180/Octoxynol-40/TMMG Copolymer; PEG-150 Pentaerythrityl Tetrastearate; PEG/PPG-120/10 Trimethylolpropane Trioleate Octyldodecyl/PPG-3 Myristyl Ether Dimer Dilinoleate; PEG-18 Castor Oil Dioleate; PEG-150/Decyl Alcohol/SMDI Copolymer; PEG-12 Dimethicone Crosspolymer; PEG-150/Stearyl Alcohol/SMDI Copolymer; PEI-7; PEI-10; PEI-15; PEI-30; PEI-35; PEI-45; PEI-250; PEI-275; PEI-700; PEI-1000; PEI-1400; PEI-1500; PEI-1750; PEI-2500; PEI-14M; Pentafluoropropane; Perfluorononyl Octyldodecyl Glycol Meadowfoamate; Phosphonobutanetricarboxylic Acid; Polyacrylamidomethylpropane Sulfonic Acid; Polyacrylate-3; Polyacrylate-10; Polyacrylate-11; Polyacrylic Acid; Polycaprolactone; Polycyclopentadiene; Polyester-5; Polyether-1; Polyethylacrylate; Polyethylene/Isopropyl Maleate/MA Copolyol; Polyglycerin-20; Polyglycerin-40; Polyglyceryl-3 Disiloxane Dimethicone; Polyglyceryl-3 Polydimethylsiloxyethyl Dimethicone; Polyglyceryl-4 Isostearate/Laurate; Polyhydroxystearic Acid; Polymethacrylic Acid; Polyoxymethylene Cyanoguanidine Urea; Polyperfluoroethoxymethoxy PEG-2 Phosphate; Polyquaternium-52; Polyvinyl AlcoholPotassium Alginate; Polyvinyl Imidazolinium Acetate; Polyvinyl Methyl Ether; Potassium Aluminum Polyacrylate; Potassium Carbomer; Potassium Polyacrylate ; PPG-3 Myristyl Ether Neoheptanoate; PPG-14 Laureth-60 Hexyl Dicarbamate; PPG-14 Laureth-60 Isophoryl Dicarbamate; PPG-14 Palmeth-60 Hexyl Dicarbamate; PVM/MA Copolymer; PVP; PVP/VA/Itaconic Acid CopolymerPEG-150/Stearyl Alcohol/SMDI Copolymer; PVP/Decene Copolymer; Ricinoleic Acid/Adipic Acid/AEEA Copolymer; Silica; Silica Dimethicone Silylate; Silica Dimethyl Silylate; Silica Silylate; Sodium Acrylates/Acrolein Copolymer; Sodium Acrylates/Acrylonitrogens Copolymer; Sodium Acrylates Copolymer; Sodium Acrylates Crosspolymer; Sodium Acrylate/Sodium Acrylamidomethylpropane Sulfonate Copolymer; Sodium Acrylate/Sodium Acryloyldimethyl Taurate/Acrylamide Copolymer; Sodium Acrylate/Sodium Acryloyldimethyl Taurate Copolymer; Sodium Acrylates/Vinyl Isodecanoate Crosspolymer; Sodium Acrylate/Vinyl Alcohol Copolymer; Sodium Acrylic Acid/MA Copolymer; Sodium Acryloyldimethyl Taurate/Acrylamide/VP Copolymer; Sodium Carbomer; Sodium C4-12 Olefin/Maleic Acid Copolymer; Sodium Glycereth-1 Polyphosphate; Sodium Isooctylene/MA Copolymer; Sodium Magnesium Fluorosilicate; Sodium Polyacrylate; Sodium Polyacrylate Starch; Sodium Polyacryloyldimethyl Taurate; Sodium Polymethacrylate; Sodium Polystyrene Sulfonate; Sodium Styrene/Acrylates Copolymer; Sodium Tauride Acrylates/Acrylic Acid/Acrylonitrogens Copolymer; Steareth-60 Cetyl Ether; Steareth-100/PEG-136/HDI Copolymer; Stearyl/PPG-3 Myristyl Ether Dimer Dilinoleate; Stearylvinyl Ether/MA Copolymer; Styrene/Acrylates/Acrylonitrile Copolymer; Styrene/Acrylates/Ammonium Methacrylate Copolymer; Styrene/MA Copolymer; TEA-Carbomer; Tosylamide/Epoxy Resin; Tosylamide/Formaldehyde Resin; Tribenzoyl Triricinolein; Tromethamine Acrylates/Acrylonitrogens Copolymer; VP/Dimethylaminoethylmethacrylate Copolymer; VP/Eicosene Copolymer; VP/Hexadecene Copolymer; VP/VA Copolymer.

In one embodiment the composition comprises an opacifier and/or pearlizer. These are known by the one skilled in the art. It is mentioned that some of these compounds, such as EGDS, can also be used as rheology modifiers. Opacifying and Pearlizing Agents are ingredients deliberately added to cosmetic products to reduce their clear or transparent appearance, and provide some shine and pearlescent appearance.

Examples of opacifiers and/or pearlizers that can be used include (INCI names): Acrylates/PEG-10 Maleate/Styrene Copolymer; Allyl Methacrylates Crosspolymer; Alumina; Aluminum Hydroxide; Aluminum Silicate; Aluminum Zinc Oxide; Arachidic Acid; Attapulgite; Barium Sulfate; Behenamide; Behenic Acid; Bentonite; Calamine; Calcium Carbonate; Calcium Silicate; Calcium Sulfate; Calcium Sulfate Hydrate; Cellulose Succinate; Cerium Oxide; Cetearyl Alcohol; Cetyl Alcohol; Chalk; Charcoal Powder; Corchorus Capsularis Powder; Corn Acid; DEA-Styrene/Acrylates/DVB Copolymer; Diatomaceous Earth; Dicalcium Phosphate; Dicalcium Phosphate Dihydrate; Dolomite; Erucamide; Fuller's Earth; Glycol Dibehenate; Glycol Dioleate; Glycol Distearate; Glycol Ditallowate; Glycol Hydroxystearate; Glycol Montanate; Glycol Palmitate; Glycol Stearate; Gossypium Herbaceum (Cotton) Powder; Guanine; Hectorite; Hydrated Silica; Hydrogenated Coconut Acid; Hydrogenated Menhaden Acid; Hydrogenated Palm Acid; Hydrogenated Tallow Acid; Hydrogenated Tallow Amide; Hydroxyethyl Acrylate/Sodium Acryloyldimethyl Taurate Copolymer; Hydroxyethyl Stearamide-MIPA; Hydroxystearyl Cetyl Ether; Iron Powder

Kaolin; Lauryl Laurate; Linoleamide; Lithium Stearate; Magnesium Aluminum Silicate; Magnesium Carbonate; Magnesium Oxide; Magnesium Silicate; Magnesium Trisilicate; Mica; Montmorillonite; Moroccan Lava Clay; Myristic Acid; Nephrite Powder; Nylon-6; Nylon 6/12; Nylon-11; Nylon-12; Nylon-66; Palm Acid; Palmitic Acid; Palm Kernel Acid; PEG-3 Distearate; PEG-4 Distearyl Ether; Pisum Sativum (Pea) Starch; Polyacrylate-4; Polyacrylate-10; Polyacrylate-11; Polydodecanamideaminium Triazadiphenylethenesulfonate; Polymethylsilsesquioxane; Polyphenylsilsesquioxane; Polyquaternium-62; Polysilicone-12; Potassium Cellulose Succinate; Propylene Glycol Distearate; Pueraria Lobota Starch; Pyrophyllite; Rubber Latex; Sapphire Powder; Sasa Senanensis Leaf Powder; Silica; Silicon/Titanium/Cerium/Iron Oxides; Silicon/Titanium/Cerium/Zinc Oxides; Sodium Acrylate/Sodium Acryloyldimethyl Taurate/Acrylamide Copolymer; Sodium Acrylate/Sodium Acryloyldimethyl Taurate Copolymer; Sodium Acryloyldimethyl Taurate/Acrylamide/VP Copolymer; Sodium Magnesium Fluorosilicate; Sodium Methacrylate/Styrene Copolymer; Sodium Styrene/Acrylates/Divinylbenzene Copolymer; Sodium Styrene/Acrylates/PEG-10 Dimaleate Copolymer; Sodium Styrene/PEG-10 Maleate/Nonoxynol-10 Maleate/Acrylates Copolymer; Stearamide; Stearamide DEA-Distearate; Stearamide DIBA-Stearate; Stearamide MEA-Stearate; Stearyl Palmitate; Styrene/Acrylamide Copolymer; Styrne/Acrylates Copolymer; Styrene/Acrylates/Acrylonitrile Copolymer; Styrene/Butadiene Copolymer; Styrene/Isoprene Copolymer; Styrene/Methylstyrene/Indene Copolymer; Styrene/VA Copolymer; Talc; Tallow Amide; Tetradecyloctadecyl Behenate; Tetradecyloctadecyl Myristate; Tetradecyloctadecyl Stearate; Tin Oxide; Titanium Dioxide; Titanium Hydroxide; Titanium Isostearates; Titanium/Titanium Dioxide; Tricalcium Phosphate; Trimagnesium Phosphate; Zinc Carbonate; Zinc Ricinoleate; Zirconium Dioxide; Zirconium Silicate

In one embodiment the composition comprises an anti-dandruff agent. These are known by the one skilled in the art. Examples of useful agents that can be used include: Asarum Heterotropoides Extract; Betula Ermanii Stem Extract; Brassica Campestris (Rapeseed) Flower Extract; Capsicum Annuum Fruit Extract; Carpinus Tschonoskii Leaf Extract; Citronellic Acid; Coal Tar; Coptis Japonica Extract; Fragaria Ananassa (Strawberry) Seed Oil; Hydroxypropyl Bisstearamide MEA; Juniperus Communis Sprout Extract; Lactobacillus/Rice ; Bran/Saccharomyces/Camellia Sinensis Leaf; Extract Ferment; Lactococcus/Bean Seed Extract Ferment Filtrate; Larix Sibirica Wood Extract; Leuconostoc/Radish Root Ferment Filtrate;

Magnesium/Aluminum/Zinc/Hydroxide/Carbonate; Mallotus Japonicus Leaf Extract; Morus Bombycis Extract; Octadecenedioic Acid; Peumus Boldus Leaf Oil ; Physocarpus Amurensis Stem Extract; Phytosphingosine Acetamide; Pinus Pinaster Bark/Bud Extract; Piroctone Olamine; Climbazole; Pogostemon Cablin Leaf/Stem Extract; Salicylic Acid; Sciadopitys Verticillata Root Extract; Selenium Sulfide; Smilax China Extract; Sodium Citronellate; Sorbus Aucuparia Seed Oil; Sulfur; Undecylenamidopropyl PEG-2 Dimonium Undecylenate; Vitis Vinifera (Grape) Seed Extract; Zinc Pyrithione (Pyrithione Zinc) ; Zinc Thiosalicylate.

Other useful further ingredients that can be used include:

- Preservatives,
- Perfumes,
- Fragrances,

- Coloring agents,
- photoprotective agents, UV filters
- emollients
- vegetal oils,
- pH regulating or adjusting agents, such as citric acid or NaOH
- foam boosting agent
- non aqueous solvents, such as ethanol, isopropanol.

Process of preparing the composition

The composition can be prepared by any suitable process. Such processes are known by the one skilled in the art. Typical processes involve mixing the different ingredients, with optionally pre-mixing some of them and/or with preparing stock solutions and/or pre-dispersions or the like. Mixing can be preformed with appropriate shear rate. Applying some significant shear might be particularly useful to obtain structured surfactants.

The structured surfactant composition can be made for example by combining and mixing an anionic surfactant and water, adding a structuring agent, then adding a liquid carrier such as water, and optionally, adjusting the pH and/or adding a preservative. Alternately the liquid carrier such as water may be added with the anionic surfactant and the water. The structured surfactant composition can also be subjected to high shear mixing. As used herein, the term "high shear mixing" refers to mixing under high shear conditions, typically at a shear rate of greater than or equal to about $1,000 \ s^{-1}$, more typically greater than or equal to about $3,500 \ s^{-1}$. The oil can be admixed before or after mixing the surfactant(s). It can be admixed before or after adjunction of an electrolyte. It can be admixed before or after high shear mixing.

Process of use of the composition

The composition is typically used to treat hair. The hair treatment is typically preformed by applying the composition onto hair, preferably onto wet hair. After application the hair is usually rinsed. The hair treatment typically provides deposition of the oil onto the hair. With the composition of the invention the hair treatment improves the hair treatment by deposition of the oil onto hair.

According to a preferred embodiment the treated hair comprises damaged hair, and optionally virgin hair. According to a preferred embodiment the treated hair comprises damaged hair and virgin hair. This embodiment encompasses the following situations:

- a part of the hair fiber in damaged (damaged part of hair fiber) and another part is not (virgin part of hair fiber), and/or
- some hair fibers are damaged (damaged hair fibers) and some hair fibers are not (virgin hair fiber).

As used herein virgin hair refers to non-damaged hair. Damages of hair can typically be observed by microscopy, preferably with a Scanning Electron Microscope. Typical damaged hair presents with such observation:

- scales, typically standing up scales
- porosity
- discoloration, and/or
- double ends.

Damaged hair can also be characterized by hydrophilic properties, with a contact angle being of lower than 90° for example.

Damage to hair can be the consequence of different causes, including chemical causes, physical causes or other causes.

One common cause of damaged hair can be linked to how one regularly treats its hair, particularly if one uses harsh chemicals. Perms, relaxers, color treatments, and bleach can all be contributing causes of damaged hair. Some of these chemicals cause the cuticles of hair strands to swell, which eventually leaves hair with scales that are rough and apt to chipping or falling off all together. Other chemicals, like perms, relaxers and bleaches are perhaps the more severe causes of damaged hair as they destroy one hair's protein bonds, which weaken the internal structure of the strands. Pollution can also be a cause of damaged hair.

Over-grooming hair or using a rough brush or comb can is perhaps one of the most common physical causes of damaged hair. Pillowcases, salt remaining on the hair after an intense workout or swim, and hair accessories can also be physical causes of damaged hair.

Sun rays and heat from hair styling instruments are also common causes of damaged hair. While the sun won't really negatively affect healthy hair, chemically processed hair doesn't respond too well to ultraviolet rays. The sun is often one of the causes of damaged hair because it can cause the hair's protein bonds to diminish and weaken. When heated styling instruments such as curling irons and blow dryers are used too often or on settings that are too hot, they can serve as causes of damaged hair.

The composition of the invention can allow improving the ratio
of treatment of damaged hair / treatment of virgin hair.

This ratio is typical of an improved selectivity of the treatment to damaged hair, or of a treatment targeting damaged hair. The selective and/or targeted treatment can be emphasized on communication tools used by suppliers of chemical ingredients of shampoo compositions, for example on animations or movies, presentations, leaflets, flyers, posters, technical data sheets, formularies, on any support, including on papers and websites. This can be linked to a complete or semi complete composition, or to a particular product used to prepare a composition, for example to a product providing a structured surfactant system and/or to a cationic co-surfactant and/or to an oil. The selective and/or targeted treatment can as well be emphasized on communication tools used in marketing shampoo compositions, for example on commercial claims, labels, documentation linked to the composition, commercials, scientific studies backing commercial claims, on any support, including on papers, labels, websites, films or animation. Examples of commercial claims can include selective or targeted deposition, selective or targeted treatment, selective or targeted hair repair or the like. Films or animation can for example show a hair fiber (or a representation thereof) having a damaged parts (for example split end or scales) and a product (or a representation thereof) approaching to the hair fiber and treating the damaged part while substantially ignoring the virgin part.

The composition can allow a better targeting of damaged hair. For example it can provide an enhanced deposition of the oil where it is most needed, for example onto tip and end regions. The improved selectivity to damaged hair can allow avoiding drawbacks of intensive and repeated deposition where it is not need, such as build-up effect and/or perception of greasy hair, typically at the root, or perception of heavy hair or dull hair.

The composition of the inventive can provide various treatment benefits such as, take-out, spreadability, rinseability, skin feeling after rinsing, combability of wet hair, feel of wet hair, combability of dry hair and/or feel of dry hair.

### Examples

**Example 1: Shampoos having a structured surfactant system and silicone oil - Effect of cationic surfactant on viscosity**

The two following structured formulations are prepared (identified as "structured-A" and "structured-B"). The formulations comprise:

- a structured surfactant system based on Miracare® SLB365 (Rhodia) surfactant blend having sodium trrideceth sulfate, disodium lauroamphoacetate and cocamide MEA, (approximately 50% surfactants in weight),
- a conditioning polymer: Jaguar@ C17 (Rhodia)
- a micronic silicone emulsion: Mirasil® DME-2 (Rhodia) (50% silicone by weight).

Formulation "Structured-A" is a comparative formulation corresponding to a recipe free of cationic surfactant.
Formulation "Structured-B" is a formulation comprising cetrimonium chloride ("CTAC") cationic surfactant: Incroquat® CTC-30 (Croda) (30% CTAC in weight).
Both formulations are detailed in Table 1 below (the amounts are the amounts in wt% "as is" as opposed to amounts as active matter).

Table 1

| | Shampoo « Structured A » Comparative | shampoo « Structured B» |
|---|---|---|
| dionized water | 28.745 | 33.245 |
| Jaguar C17 | 0.35 | 0.35 |
| Versene 100 | 0.05 | 0.05 |
| Miracare SLB365 | 45 | 45 |
| Incroquat CTC-30, 30wt% active | 0 | 3 |
| Mirasil DME-2 | 6.48 | 6.48 |
| NaCl solution, 20wt% active | 17.5 | 10 |

(continued)

| | Shampoo « Structured A » Comparative | shampoo « Structured B» |
|---|---|---|
| **Sodium benzoate** | 0.5 | 0.5 |
| **Citric acid, 50wt% active** | 1.375 | 1.375 |
| **Viscosity** | 18000 cP | 21080 cP |

The <u>procedure</u> for preparing 100g of formulations Structured-A and Structured-B is the following:

- mix the required amount of dionized water together with Jaguar C17, so that the end concentration of the polymer is the same (0.35% in weight) as that in the SSL shampoo formulations of Table 1;
- adjust the pH of the solution with the required amount of Versene 100 (EDTA);
- add the required amount of Miracare SLB365 so that the final concentration of this blend is 45% in weight in the formulation;
- add the required amount of Incroquat CTC-30 so that the final concentration of CTAC is 0 or 0.9% in weight in the formulation;
- add the required amount of Mirasil DME-2 so that the final concentration in silicone oil is 3% in weight (solids) in the formulation.
- add the required amount of a sodium chloride solution (20% NaCl in weight) so that the final salt concentration in NaCl ranges between 2.0 and 3.5% in weight in the formulation, depending on whether Incroquat CTC-30 has been added or not;
- add 0.5g of Sodium Benzoate preservative; adjust the pH of the solution to pH=6 to 6.5 with a concentrated citric acid solution (50% citric acid in weight).

<u>Evaluation</u>

The viscosity of both formulations is measured at room temperature, after a 24 hour resting time, using a Brookfield rheometer (spindle RV4, viscosity taken at 10rpm). The viscosity is reported on table 1. Formulation "Structured-B" presents a higher viscosity with a substantially lower amount of salt. The cationic surfactant can thus be used in a structure surfactant system to lower the amount of salt and thus to reduce potential irritancy.

**<u>Comparative Example 2</u> Shampoos having surfactants in micellar form and a silicone oil.**

As reference comparative examples, the four following micellar formulations ("micellar A", "micellar B", "micellar C", "micellar D") are prepared. The formulations comprise:

- sodium laurylethersulfate ("SLES"), Empicol® ESB3/M (Huntsman) (27.2% SLES in weight)
- cocamidopropylbetaine ("CAPB"), Mirataine® BETC30 (Rhodia) (30% CAPB in weight)
- a conditioning polymer: Jaguar® C17 (Rhodia)
- various silicone emulsions:

    - a micronic silicone emulsion: Mirasil® DME-2 (Rhodia) (50% silicone by weight), or
    - a submicronic silicone emulsion: Mirasil® DME-0.6 (Rhodia) (65% silicone in weight)
    - in some shampoos: cetrimonium chloride ("CTAC") <u>cationic surfactant</u>: Incroquat® CTC-30 (Croda) (30% CTAC in weight)

The formulations are detailed in Table 2 below (the amounts are the amounts in wt% "as is" as opposed to amounts as active matter).

Table 2

| | micellar shampoo A | micellar shampoo B | micellar shampoo C | micellar shampoo D |
|---|---|---|---|---|
| **dionized water** | 35.3 | 32.3 | 34 | 31 |
| **Jaguar C17** | 0.35 | 0.35 | 0.35 | 0.35 |
| **Mirataine BETC30** | 6.65 | 6.65 | 6.65 | 6.65 |

(continued)

| | micellar shampoo A | micellar shampoo B | micellar shampoo C | micellar shampoo D |
|---|---|---|---|---|
| **Empicol ESB/3M** | 51.4 | 51.4 | 51.4 | 51.4 |
| **Incroquat CTC-30** | 0 | 3 | 0 | 3 |
| **Khaton CG** | 0.1 | 0.1 | 0.1 | 0.1 |
| **NaCl** | 1.6 | 1.6 | 1.6 | 1.6 |
| **Mirasil DME-0.6** | 4.6 | 4.6 | 0 | 0 |
| **Mirasil DME-2** | 0 | 0 | 5.9 | 5.9 |

The procedure for preparing 100g of formulation is the following:

- mix the required amount of dionized water together with Jaguar C17, so that the end concentration of the polymer is the same (0.35% in weight) as that in the SSL shampoo formulations of Table 1;
- adjust the pH of the solution to pH=4 to 5 with the required amount of a concentrated solution (50% in weight) of citric acid;
- add the required amount of Mirataine BETC30 so that the final concentration of CAPB is 2% in weight in the formulation;
- add the required amount of Empicol ESB3/M so that the final concentration of SLES is 14% in weight in the formulation;
- add the required amount of Incroquat CTC-30 so that the final concentration of CTAC is 0 or 0.9% in weight in the formulation;
- add 0.1g of Kathon CG preservative; adjust the pH of the solution to pH=6 to 6.5;
- add sodium chloride (NaCl) so that the final salt concentration is 1.6% in weight in the formulation;
- add the required amount of Mirasil DME-0.6 or Mirasil DME-2 so that the final concentration in silicone oil is 3% in weight (solids) in the formulation.

Micellar shampoos C and D of comparative example 2 are based on the same silicone oil emulsion (Mirasil® DME-2) can be directly compared to structures shampoos A and B of Example 1.

### Example 3: Evaluation of silicon oil-deposition on virgin & damaged hair and evaluation of selectivity

The evaluation involves structured and micellar shampoos listed in Tables 1 and 2, containing either 0 or 3% of Incroquat CTC-30 in the final formulation, and in all cases 3wt% of silicone oil coming from two types of silicone emulsions, either submicronic (DME0.6) or micronic (DME2).

Silicone oil deposition is evaluated on virgin hair virgin as well as on damaged hair with each formulation, by washing using 10 grams of hair approximately with dose of shampoo of 1 gram. The silicone oil deposited on hair is extracted with tetrahydrofuran (THF) and the deposition yield is measured with gas chromatography. The results are given in Table 3 below.

From the deposited amounts one defines the deposition selectivity, by taking the ratio of the amount of silicone deposited on damaged hair, to that deposited on virgin hair:

$$\text{selectivity} = \frac{\text{amount deposited on damaged hair}}{\text{amount deposited on virgin hair}} \qquad \text{Eq. 1}$$

Table 3

| Shampoo reference | VIRGIN HAIR | | DAMAGED HAIR | | selectivity |
|---|---|---|---|---|---|
| | deposition yield (%) | ppm silicone µg/g of hair | deposition yield (%) | ppm silicone µg/g of hair | |
| Micellar shampoo A (comparative) | $56.8 \pm 6.7$ % | $1963 \pm 208$ | $5.2 \pm 1.9$% | $157 \pm 56$ | $0.08 \pm 0.019$ |

(continued)

| Shampoo reference | VIRGIN HAIR | | DAMAGED HAIR | | selectivity |
|---|---|---|---|---|---|
| | deposition yield (%) | ppm silicone μg/g of hair | deposition yield (%) | ppm silicone μg/g of hair | |
| Micellar shampoo B (comparative) | $37.5^{\pm0.4}$ % | $1306^{\pm9}$ | $1.7^{\pm0.5}$ % | $51^{\pm14}$ | $0.04^{\pm0.005}$ |
| Micellar shampoo C (comparative) | $14.3^{\pm0.1}$ % | $490^{\pm0}$ | $1.7^{\pm06}$ % | $50^{\pm17}$ | $0.10^{\pm0.035}$ |
| Micellar shampoo D (comparative) | $7.9^{\pm0.1}$ % | $273^{\pm3}$ | $1,5^{\pm0.9}$ % | $45^{\pm26}$ | $0.16^{\pm0.049}$ |
| Structure shampoo A (comparative) | $14.1^{\pm16}$ % | $527^{\pm49}$ | $3.3^{\pm1.3}$ % | $100^{\pm37}$ | $0.19^{\pm0.045}$ |
| Structure shampoo B | $29.7^{\pm0.0}$ % | $938^{\pm36}$ | $11,9^{\pm1.8}$ % | $354^{\pm47}$ | $0.38^{\pm0.032}$ |

As to deposition

Regardless of the size of the silicone oil, the deposition yield of micellar shampoos collapses when the cationic surfactant CTAC is added, and this is true on virgin as well as damaged hair. For instance, with the finest submicronic emulsion Mirasil® DME-0.6 it is found that:

- On virgin hair, the deposition of silicone is of $1963^{\pm208}$ ppm with Micellar shampoo A and of $1306^{\pm9}$ ppm with Micellar shampoo B which further contains CTAC, implying a reduction of -33% of the deposited amount of the submicronic silicone emulsion Mirasil® DME-0.6;
- On damaged hair, the deposition of silicone is of $157^{\pm56}$ ppm with Micellar shampoo A and of $51^{\pm14}$ ppm with Micellar shampoo B which further contains CTAC, implying a reduction of -68% of the deposited amount of the finest submicronic emulsion Mirasil® DME-0.6.

Similar collapses in silicone oil deposition are found with the micronic silicone oil Mirasil DME-2 in micellar shampoos added with cationic surfactant CTAC.
On the contrary, adding cationic surfactant CTAC to structured shampoos improves the efficiency in silicone oil deposition on both types of hair:

- On virgin hair, the deposition of silicone is of $527^{\pm49}$ ppm with Structured shampoo A and of $938^{\pm36}$ ppm with Structured shampoo B which further contains CTAC, implying an increase of +78% of the deposited amount of silicone oil;
- On damaged hair, , the deposition of silicone is of $100^{\pm37}$ ppm with Structured shampoo A and of $354^{\pm47}$ ppm with Structured shampoo B which further contains CTAC, implying an increase of +72% of the deposited amount of silicone oil.

In all cases, the amounts deposited are much larger than those achieved by Micellar shampoos C and D, which are based on the same silicone oil (Mirasil DME-2).

As to selectivity

The selectivity of micellar shampoos is poor, whether the cationic surfactant CTAC is added or not, and regardless of the size of the silicone emulsion,: micellar shampoos in all cases achieve a deposition ratio on damaged-to-virgin hair of about 0.1 (see Table 3): the actual selectivity numbers range from $0.04^{\pm0.005}$ for Micellar Shampoo B to $0.16^{\pm.0.009}$ for Micellar shampoo D. In other words, micellar shampoos deposit silicone about 10 times less on damaged than on virgin hair.
The selectivity of structured shampoos is much larger: Structured shampoo A has a selectivity of $0.19^{\pm0045}$, while Structured shampoo B which contains 0.9% in weight of the cationic surfactant CTAC, has a selectivity twice as large, of $0.38^{\pm0.032}$. Thus the presence of cationic surfactant in a structured shampoo boosts the selectivity.
This allows a significant treatment of damaged parts of hair while avoiding excess of treatment on virgin parts of hair and can allow reducing build-up negative effects.

### Example 4: Sensorial Performance Evaluation (consumer test)

Structured shampoo A (comparative, free of CTAC, see Table 1) and structured shampoo B (comprising CTAC, see table 1) are evaluated by an independent specialized Institute (Institut Dr. Schrader - Creachem GmbH - Max-Planck-Straße 6 - 37603 Holzminden).

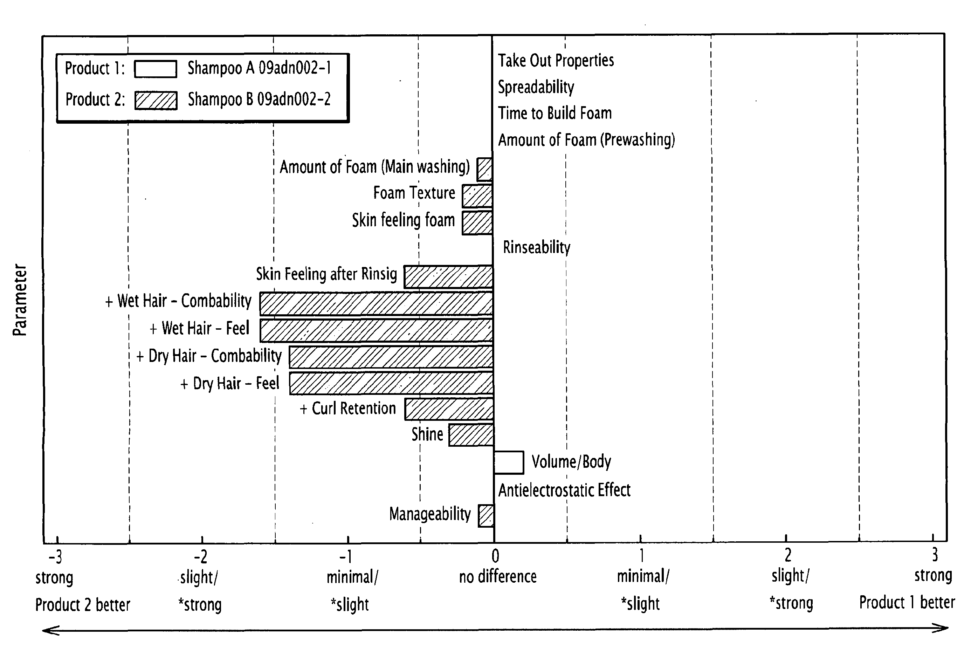

According to this evaluation half-head tests are performed on 10 volunteers with middle-long dry/damaged hair: Shampoo A is applied (according to standard a hair cleaning procedure - 1 shampoo application) on one side of the head and Shampoo B is applied on the other side of the head. Application is performed by a trained expert. The two shampoos are directly compared by expert in terms of: (1) take-out property, (2) spreadability, (3) time to build foam, (4) amount of foam, (5) foam texture, (6) skin feel foam, (7) rinseability, (8) skin feeling after rinsing, (9) combability of wet hair, (10) feel of wet hair, (11) combability of dry hair, (12) feel of dry hair. Results are reported in Figure 1.

Figure 1 presents the comparison of the performances of Structured shampoos A (reported as "Product 1") and B (comparative - reported as "Product 2").

After only one application, Structured Shampoo B (Product 2) shows a statistical improvement on all attributes associated with hair combability and hair feel, in wet as well as dry conditions.

Structured Shampoo B (Product 2) also brings a statistical improvement in curl retention compared to the Structured Shampoo A (comparative - Product 1).

Structured Shampoo B (Product 2) does not negatively impact other shampooing attributes like foam generation, foam volume, foam texture and rinseability compared to Structured Shampoo A (comparative - Product 1).

Structured Shampoo B (Product 2) does not negatively impact other hair attributes after shampooing like shine, volume body, antielectrostatic effects and manageability.

## Claims

1. A process for improving hair treatment by deposition of an oil onto hair comprising the step of applying onto hair an aqueous composition comprising:

   a) a structured surfactant system comprising:

   - non cationic surfactant(s), and
   - a cationic co-surfactant

   b) an oil, being

   b1) a silicone oil, or
   b2) an oil of mineral origin, and

   c) optionally a cationic or ampholytic polymer.

2. The process according to claim 1, wherein the structured surfactant system presents the following:

   - planar lamellar phases,
   - multilamellar vesicles phases, or
   - a mixture of planar lamellar and multilamellar vesicles phases.

3. The process according to any of the preceding claims, wherein the structured surfactant system and/or the composition has an opaque visual appearance and exhibits a yield strength of greater than 0 Pa.

4. The process according to any of the preceding claims, wherein the structured surfactant system a) comprises:

   a1) at least one cationic co-surfactant,
   a2) at least one anionic surfactant,
   a3) at least one structurant,
   a4) optionally at least one amphoteric or zwitterionic surfactant, and
   a5) optionally at least one electrolyte.

5. The process according to any of the preceding claims, wherein the cationic co-surfactant is:

- cetrimonium chloride,
- cetrimonium bromide,
- cetrimonium methosulfate
- Behentrimonium Chloride;
- Behentrimonium Methosulfate
- Stearamidopropyl Dimethylamine
- Isostearamidopropyl Dimethylamine
- Oleamidopropyl Dimethylamine
- Behenamidopropyl Dimethylamine
- Cocamidopropyl Dimethylamine Propionate
- Stearamidopropyl Dimethylamine Lactate
- Stearamidopropyl Morpholine Lactate
- Isostearamidopropyl Mospholine Lactate, or
- Sunflowerseedamidopropyl Dimethylamine.

6. The process according to any of the preceding claims, where in the composition comprises from 0.01 to 5 % by weight, preferably from 0.5 to 1% of the cationic co-surfactant.

7. The process according to any of the preceding claims, wherein the weight ratio between the cationic co-surfactant and the other surfactant(s) is of from 0.5/100 to 10/100, preferably of from 1/100 to 10/100, preferably of from 2/100 to 8/100.

8. The process according to any of the preceding claims, wherein the total amount of non cationic surfactant(s), including structurant(s) if any, is of from 10% to 25% by weight, preferably of from 13.5% to 22%, for example of from 16.5% to 19.5%.

9. The process according to any of the preceding claims, wherein the silicone oil is:

- a dimethicone
- an amodimethicone
- a dimethiconol, or
- a PEG-dimethicone.

10. The process according to any of the preceding claims, wherein the silicone oil is present in the composition in the form of:

- a microemulsion with a particle size of lower than 0.15 $\mu$m,
- an emulsion with a particle size of from 0.15 $\mu$m to lower than 1 $\mu$m, or of from 1 $\mu$m to lower than 1.5 $\mu$m or of from 1.5 $\mu$m to lower than 2 $\mu$m, or from 2 $\mu$m to lower than 2.5 $\mu$m, or from 2.5 $\mu$m to lower than 4 $\mu$m, or from 4 $\mu$m to lower than 10 $\mu$m, or from 10 $\mu$m to lower than 30 $\mu$m, or from 30 $\mu$m to 100 $\mu$m.

11. The process according to any of the preceding claims, comprising from 0.1 % by weight to 5% by weight of the oil.

12. The process according to any of the preceding claims, wherein the cationic or ampholytic copolymer is:

c1) a modified polysaccharide
c2) a synthetic cationic polymer, or
c3) a synthetic ampholytic copolymer.

13. The process according to claim 1, wherein the hair comprises damaged hair and optionally virgin hair.

14. The process according to claim 13, providing an improved ratio of:

treatment of damaged hair / treatment of virgin hair.

**Patentansprüche**

1.  Verfahren zur Verbesserung der Behandlung von Haar durch Abscheidung eines Öls auf das Haar, umfassend den Schritt der Applikation einer wässrigen Zusammensetzung auf das Haar, umfassend:

    a) ein strukturiertes Tensidsystem, umfassend:

    - (ein) nicht-kationische(s) Tensid(e), und
    - ein kationisches Cotensid

    b) ein Öl, das

    - b1) ein Silikonöl, oder
    - b2) ein Öl mineralischen Ursprungs ist, und

    c) fakultativ ein kationisches oder ampholytisches Polymer.

2.  Verfahren nach Anspruch 1, wobei das strukturierte Tensidsystem das Folgende präsentiert:

    - ebenflächige lamellare Phasen,
    - multilamellare Vesikel-Phasen, oder
    - ein Gemisch aus ebenflächigen lamellaren und multilamellaren Vesikel-Phasen.

3.  Verfahren nach einem der vorhergehenden Ansprüche, wobei das strukturierte Tensidsystem und/oder die Zusammensetzung ein opakes visuelles Aussehen aufweist und eine Fließgrenze von mehr als 0 Pa zeigt.

4.  Verfahren nach einem der vorhergehenden Ansprüche, wobei das strukturierte Tensidsystem a) das Folgende umfasst:

    a1) wenigstens ein kationisches Cotensid,
    a2) wenigstens ein anionisches Tensid,
    a3) wenigstens ein Strukturmittel,
    a4) fakultativ wenigstens ein amphoterisches oder zwitterionisches Tensid, und
    a5) fakultativ wenigstens einen Elektrolyten.

5.  Verfahren nach einem der vorhergehenden Ansprüche, wobei das kationische Cotensid:

    - Cetrimoniumchlorid
    - Cetrimoniumbromid
    - Cetrimoniummethosulfat
    - Behentrimoniumchlorid
    - Behentrimoniummethosulfat
    - Stearamidopropyldimethylamin
    - Isostearamidopropyldimethylamin
    - Oleamidopropyldimethylamin
    - Behenamidopropyldimethylamin
    - Cocamidopropyldimethylamin-propionat
    - Stearamidopropyldimethylamin-lactat
    - Stearamidopropylmorpholin-lactat
    - Isostearamidopropylmorpholin-lactat, oder
    - Sonnenblumenkern-Amidopropyldimethylamin ist.

6.  Verfahren nach einem der vorhergehenden Ansprüche, wobei die Zusammensetzung 0,01 bis 5 Gewichts-%, vorzugsweise 0,5 bis 1 % des kationischen Cotensids umfasst.

7.  Verfahren nach einem der vorhergehenden Ansprüche, wobei das Gewichtsverhältnis zwischen dem kationischen Cotensid und dem (den) anderen Tensid(en) 0,5/100 bis 10/100, vorzugsweise 1/100 bis 10/100, vorzugsweise 2/100 bis 8/100 beträgt.

**8.** Verfahren nach einem der vorhergehenden Ansprüche, wobei die Gesamtmenge an nicht-kationischen Tensid(en), einschließlich gegebenenfalls Strukturmittel, 10 Gewichts-% bis 25 Gewichts-%, vorzugsweise 13,5% bis 22%, beispielsweise 16,5% bis 19,5% beträgt.

**9.** Verfahren nach einem der vorhergehenden Ansprüche, wobei das Silikonöl:

- ein Dimethicon
- ein Amodimethicon
- ein Dimethiconol, oder
- ein PEG-Dimethicon ist.

**10.** Verfahren nach einem der vorhergehenden Ansprüche, wobei das Silikonöl in der Zusammensetzung in folgender Form vorliegt:

- als eine Mikroemulsion mit einer Partikelgröße unter $0{,}15\ \mu m$,
- als eine Emulsion mit einer Partikelgröße von $0{,}15\ \mu m$ bis unter $1\ \mu m$, oder von $1\ \mu m$ bis unter $1{,}5\ \mu m$, oder von $1{,}5\ \mu m$ bis unter $2\ \mu m$, oder von $2\ \mu m$ bis unter $2{,}5\ \mu m$, oder von $2{,}5\ \mu m$ bis unter $4\ \mu m$, oder von $4\ \mu m$ bis unter $10\ \mu m$, oder von $10\ \mu m$ bis unter $30\ \mu m$, oder von $30\ \mu m$ bis $100\ \mu m$.

**11.** Verfahren nach einem der vorhergehenden Ansprüche, umfassend 0,1 Gewichts-% bis 5 Gewichts-% des Öls.

**12.** Verfahren nach einem der vorhergehenden Ansprüche, wobei das kationische oder ampholytische Copolymer:

c1) ein modifiziertes Polysaccharid
c2) ein synthetisches kationisches Polymer, oder
c3) ein synthetisches ampholytisches Copolymer ist.

**13.** Verfahren nach Anspruch 1, wobei das Haar geschädigtes Haar und fakultativ unbehandeltes Haar umfasst.

**14.** Verfahren nach Anspruch 13, das ein verbessertes Verhältnis bereitstellt von:

Behandlung von geschädigtem Haar / Behandlung von unbehandeltem Haar.

## Revendications

**1.** Procédé d'amélioration du traitement des cheveux par dépôt d'une huile sur les cheveux, comprenant l'étape d'application sur les cheveux d'une composition aqueuse comprenant :

a) un système tensioactif structuré comprenant :

- un ou plusieurs agents tensioactifs non cationiques, et
- un co-agent tensioactif cationique,

b) une huile, qui est
b1) une huile de silicone, ou
b2) une huile d'origine minérale, et
c) éventuellement un polymère cationique ou ampholytique.

**2.** Procédé selon la revendication 1, dans lequel le système tensioactif structuré présente ce qui suit :

- des phases lamellaires planaires,
- des phases vésiculaires multilamellaires, ou
- un mélange de phases lamellaires planaires et vésiculaires multilamellaires.

**3.** Procédé selon l'une quelconque des revendications précédentes, dans lequel le système tensioactif structuré et/ou la composition possède un aspect visuel opaque et présente une limite d'élasticité supérieure à 0 Pa.

**4.** Procédé selon l'une quelconque des revendications précédentes, dans lequel le système tensioactif structuré a) comprend :

   a1) au moins un co-agent tensioactif cationique,
   a2) au moins un agent tensioactif anionique,
   a3) au moins un agent structurant,
   a4) éventuellement au moins un agent tensioactif amphotère ou zwitterionique, et
   a5) éventuellement au moins un électrolyte.

**5.** Procédé selon l'une quelconque des revendications précédentes, dans lequel le co-agent tensioactif cationique est :

   - le chlorure de cétrimonium,
   - le bromure de cétrimonium,
   - le méthosulfate de cétrimonium,
   - le chlorure de béhentrimonium,
   - le méthosulfate de béhentrimonium,
   - la stéaramidopropyldiméthylamine,
   - l'iso-stéaramidopropyldiméthylamine,
   - l'oléamidopropyldiméthylamine,
   - la béhenamidopropyldiméthylamine,
   - le propionate de cocamidopropyldiméthylamine,
   - le lactate de stéaramidopropyldiméthylamine,
   - le lactate de stéaramidopropylmorpholine,
   - le lactate d'iso-stéaramidopropylmorpholine, ou
   - l'amidopropyl diméthylamine de graine de tournesol.

**6.** Procédé selon l'une quelconque des revendications précédentes, dans lequel la composition comprend de 0,01 à 5% en poids, de préférence de 0,5 à 1% en poids du co-agent tensioactif cationique.

**7.** Procédé selon l'une quelconque des revendications précédentes, dans lequel le rapport pondéral entre le co-agent tensioactif cationique et le ou les autres agents tensioactifs va de 0,5/100 à 10/100, de préférence de 1/100 à 10/100, de préférence de 2/100 à 8/100.

**8.** Procédé selon l'une quelconque des revendications précédentes, dans lequel la quantité totale des un ou plusieurs agents tensioactifs non cationiques, y compris le ou les agents structurants le cas échéant, va de 10% à 25% en poids, de préférence de 13,5% à 22%, par exemple de 16,5% à 19,5%.

**9.** Procédé selon l'une quelconque des revendications précédentes, dans lequel l'huile de silicone est :

   - une diméthicone,
   - une amodiméthicone,
   - un diméthiconol, ou
   - un PEG-diméthicone.

**10.** Procédé selon l'une quelconque des revendications précédentes, dans lequel l'huile de silicone est présente dans la composition sous forme :

   - d'une microémulsion ayant une taille de particules inférieure à 0,15 $\mu$m,
   - d'une émulsion ayant une taille de particules allant de 0,15 $\mu$m à moins de 1 $\mu$m, ou allant de 1 $\mu$m à moins de 1,5 $\mu$m, ou allant de 1,5 $\mu$m à moins de 2 $\mu$m, ou allant de 2 $\mu$m à moins de 2,5 $\mu$m, ou allant de 2,5 $\mu$m à moins de 4 $\mu$m, ou allant de 4 $\mu$m à moins de 10 $\mu$m, ou allant de 10 $\mu$m à moins de 30 $\mu$m, ou allant de 30 $\mu$m à 100 $\mu$m.

**11.** Procédé selon l'une quelconque des revendications précédentes, comprenant de 0,1% en poids à 5% en poids de l'huile.

**12.** Procédé selon l'une quelconque des revendications précédentes, dans lequel le copolymère cationique ou ampholytique est :

    c1) un polysaccharide modifié,
    c2) un polymère cationique synthétique, ou
    c3) un copolymère ampholytique synthétique.

**13.** Procédé selon la revendication 1, dans lequel les cheveux comprennent des cheveux endommagés et éventuellement des cheveux vierges.

**14.** Procédé selon la revendication 13, fournissant un rapport amélioré de :

    traitement de cheveux endommagés/traitement de cheveux vierges.

EP 2 393 469 B1

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 586275 A
- WO 03055456 A
- WO 9705857 A
- WO 2000059454 A
- WO 01019949 A
- WO 9932069 A
- WO 0170926 A
- WO 0170193 A
- WO 0267892 A
- WO 03017968 A
- WO 2005084614 A
- WO 2002005758 A
- WO 2005063174 A
- WO 2005110355 A
- US 20080153730 A
- WO 03055455 A
- WO 2006023548 A
- WO 2006127394 A
- WO 20060135627 A
- WO 2008039440 A
- US 20080233061 A